# EUROPEAN PATENT SPECIFICATION

(11) **EP 0 681 842 B1**
(45) Date of publication and mention of the grant of the patent: **13.02.2002**
(21) Application number: 95110334.0
(22) Date of filing: 19.11.1991
(51) Int. Cl.: A61K 38/27, A61K 38/30

(54) **Pharmaceutical composition comprising growth hormone and growth hormone binding protein**
Pharmazeutische Zusammensetzung enthaltend Wachstumshormon und Wachstumshormon-Bindungsprotein
Composition pharmaceutique contenant l'hormone de la croissance et la protéine se liant à l'hormone de la croissance

(30) Priority: 19.11.1990 US 615538
(43) Date of publication of application: 15.11.1995
(62) Divisional of application: 92901281.3
(73) Proprietor: GENENTECH, INC., South San Francisco California 94080 (US)
(72) Inventor: Carlsson, Lena Mariana Susann, San Francisco, California 94122 (US); Clark, Ross Graham, Pacifica, California 94044 (US); Wong, Wai Lee Tan, Los Altos, California 94022 (US)
(74) Representative: Walton, Seán Malcolm

(56) References cited:
- EP-A- 0 369 943
- WO-A-89/08667
- WO-A-89/09792
- WO-A-90/00569
- CLEMMONS D R ET AL: "A FACTOR CONTAINED IN PLASMA IS REQUIRED FOR IGF BINDING PROTEIN-1 TO POTENTIATE THE EFFECT OF IGF-I ON SMOOTH MUSCLE CELL DNA SYNTHESIS" J CELL PHYSIOL, 145 (1). 1990. 129-135., XP002062027

## Description

### Field of the Invention

A novel ligand-mediated immunofunctional assay (LIFA) method is described for detecting the presence and quantitating the amount of a polypeptide hormone binding protein in a biological fluid and/or determining the amount of the ligand polypeptide hormone specifically bound to the hormone binding protein. This modified immunometric assay for a hormone binding protein uses: 1) a first solid phase bound antibody to capture the hormone binding protein; 2) a saturating amount of ligand hormone; and, 3) a labeled second antibody specific for the ligand hormone. The LIFA method is exemplified by growth hormone binding protein assay.

### Description of the Background Art

A hormone binding protein (HBP) is a carrier protein found in biological fluids which has binding specificity for a ligand polypeptide hormone. Examples of such HBP's are growth hormone binding protein (GHBP), epidermal growth factor (EGF) binding protein, insulin-like growth factor 1 and 2 (IGF-1, IGF-2) binding proteins (six of them), platelet derived growth factor (PDGF) binding protein, nerve growth factor (NGF) binding protein, insulin binding protein, corticotropin releasing factor (CRF) binding protein, transforming growth factor beta (TGF-β) binding protein and activin binding protein (Follistatin).

One of the best characterized polypeptide hormone binding proteins is the GHBP. The GHBP discussed in this invention is the extracellular domain of the GH receptor which circulates in blood and functions as a GHBP in several species (Ymer, S.I, and Herington, A.C., Mol. Cell. Endocrinol. 41:153 [1985]; Smith, W.C. and Talamantes, F., Endocrinology 123:1489-94 [1988]; Emtner, M., and Roos, P., Acta Endocrinologica [Copenhagen] 122:296-302 [1990]), including man (Baumann, G. *et al.*, J. Clin. Endocrinol. Metab. 62:134-141 [1986]; Herington, A.C. *et al.,* J. Clin. Invest. 77:1817-1823 [1986]). Little is known about the fate of the GHBP or its regulation in various physiological and pathological conditions.

EP 0369 943 A describes the cloning of an IGF binding protein and its therapeutic applications, either alone or in combination with IGF.

Hormone binding proteins have been assayed by antibody based precipitation methods where the ligand is labeled and the antibody Is specific for the binding protein itself. Monoclonal antibodies specific for human growth hormone binding protein (hGHBP) were used by Barnard *et al*., J. Endocrinol, 123(2):327-32[1989]; for rabbit GHBP by Ymer *et al.,* Endocrinology, 125(2):993-9[1989]; and mouse by Smith *et al.*, Endocrinology 123(3):1489-94[1988]. Currently available methods for estimating GHBP levels in blood are based on incubation of the sample with radiolabeled GH, followed by separation of bound and free GH (Baumann, G. *et al*., Acta Endocrinologica (Copenhagen) 119: 529-34 [1988]; Amit, T. *et al.*, J. Clin. Endo. Metab. 71:474-479 [1990]). The results obtained by these assays are difficult to interpret due to interference by endogenous GH (Baumann, G. *et al*., J. Clin. Endocrinol. Metab. 62:134-141 [1986]). Others who have used labeled growth hormone to detect GHBP are: Emtner *et al.,* Acta Endocrinol 122(3):296-302, (1990); Silbergeld *et al*., Clin. Endocrinol. 31(3):295-303 (1989); Daughaday *et al*., J. Clin. Endocrinol Metab., 65(5):1072-4, (1987); Herington *et al*., J. Clin. Invest. 77(6):1817-23, (1986); and Laron *et al*., Acta Endocrinol. 121(4):603-8 (1989). These assays for GHBP in blood have serious problems. They are laborious, requiring separation of complexed GHBP-GH by size-exclusion chromatography or antibody precipitation, and they may not give consistent results from one laboratory to another. In addition, they generate results that are arbitrary (i.e. not calibrated to a common protein standard) and influenced by endogenous growth hormone. Therefore, there is a need for an improved assay method which will allow detection of all the polypeptide hormone binding proteins, including those bound to endogenous polypeptide hormone.

Methods for the production of monoclonal antibody-producing hybridomas are disclosed by Kipps and Herzenberg in Clinical Endocrinology and Metabolism, Vol 62, No. 1, page 108.1-108.9 (1986)

A monoclonal antibody-based immunoradiometric assay for IGF binding protein was described by Pekonen *et al*, J. Immunoassay 10:325-37 (1989). Immunometric or sandwich immunoassays using high affinity monoclonal antibodies were taught in David *et al.*, U.S. Pat. 4,486,530. Such sandwich assays have an antigen with two or more epitopes sandwiched between two antibodies. Circulating proteins that bind non-polypeptide hormone ligands, such as the thyroxine binding protein, have been measured using fluorescent labeled tracer (U.S. Pat. 4,476,228) in order to determine the number of binding protein sites not occupied by thyroxine. Iodothyronine immunoassays in a biological fluid using blocking agents and thyroxine binding globulin were described in Gordon *et al*, U.S. Pat. 4,622,293.

Specific binding pairs (SBP) are discussed in reference to antigen-antibody reactions, ligand-receptor, hormone-receptor and lectin-oligosaccharide (U.S. Pat. 4,956,302). Zuk *et al.*, (U.S. Pat. 4,594,327) describes assays of whole blood to detect members of such SBPs wherein one member of the SBP must be attached to the solid phase prior to contacting the blood. The other second member of the SBP is detected using labeled second SBP member in competitive reactions. Similarly, Weng *et al.* (U.S. Pat. 4,737,456) describes a method of reducing interfering substances in assays of a SBP member wherein the individual member of the SBP is labeled. The receptor is used in a competitive assay to capture both labeled and unlabeled ligand, not to analyze for the presence of and quantify receptor as in the present invention. A method for the determination of an antigen using two antibodies is disclosed in U.S. Pat. 4,343,896.

Problems in detecting HBP can be best illustrated by the problems encountered in detecting GHBP. Previous methods for determining the presence of GHBP in biological fluids were not as accurate as desired and frequently required the use of radioactive materials. The present assay avoids the problems of the previous assay methods in that it (a) does not require the use of radiolabeled GH; (b) does not require the removal of endogenous GH from the GHBP; (c) does not require any form of size separation of GH from the GH-GHBP complex; and, (d) measures the actual mass or absolute amount of GHBP rather than a relative amount reported in arbitrary units. The present assay has the added advantage that it measures the binding capacity of the circulating GHBP and is able to measure the degree of saturation of the GHBP with respect to GH. Moreover, the present assay is specific for GHBP by substantially reducing background assay noise that causes imprecision. The assay avoids the problems in standard immuno-metric assays by using a first antibody to capture the GHBP and a second detectably labeled antibody to measure the presence of bound GH. The use of a second antibody specific for another epitope on the GHBP would not determine whether the GHBP was functional, and in addition, it could increase the background due to other serum proteins which bound both antibodies.

In order to study the function of the endocrine system it is essential to have access to reliable methods for quantitation of all parts of the system, i.e. the hormone, its binding protein and the hormone-binding protein complex. These measurements have not been previously achieved because of interference in the assays by the different components and the fact that both the hormone and the BP can be present in free and complexed forms. It is even more complicated when there are several different binding proteins for the same hormone, as for the IGF-1 system. However, the present assay teaches how the use of monoclonal antibodies directed at a specific binding protein can measure the amount and degree of saturation of that specific binding protein. In the case of GH, for which a second binding protein with lower affinity has been described (Baumann, G. and Shaw, M.A., J. Clin. Endocrinol. Metab. 70:680-686 [1990]), this binding protein appears to be structurally unrelated to the GH receptor and should not be detected in our assay. However, this other GH binding protein may also be detected in the present assay method once the other binding protein is isolated and appropriate antibodies are raised.

Currently, the standard method for quantitation of carrier proteins for peptide hormones is incubation of serum with the radiolabeled ligand, followed by chromatography or precipitation to separate the bound and free hormone. These procedures are laborious and the results often difficult to interpret because of the interference by endogenous hormone in the sample. These assay methods give an estimate of the binding capacity of serum proteins of a certain size but the activities of different binding proteins of similar size are not distinguished and the relative proportion of free and complexed BP cannot be determined. Another disadvantage is that the results are expressed in relation to reference serum pools, which makes it difficult to compare the results in different studies. We developed an assay for the GHBP by choosing a new approach. This assay was surprisingly able to precisely detect individual hormone binding proteins in a way that has not been previously demonstrated.

The LIFA, is simple to use and has the advantage that only functional binding protein is detected. When the assay method is applied to GHBP, both total and endogenously complexed GHBP are measured, and the assay does not require removal of endogenous GH from the GHBP or procedures to separate free GH from the GHBP complex. In contrast to previous methods, endogenous GH does not interfere in the assay; instead, bound GH, either endogenous or exogenously added, is used to detect the total and complexed GHBP. In fact, one cannot use the GH as the first member bound to the solid phase since GH cannot complex with binding protein that is already complexed with endogenous GH. Therefore, one requirement of the present assay method is for a solid phase coat antibody which recognizes the binding protein both in a free and complexed form. The assay method can also be used to measure the total binding capacity and the saturation of other polypeptide hormone-binding proteins with respect to the ligands that they bind.

### SUMMARY OF THE INVENTION

The therapeutic use of GHBP alone and in combination with GH to stimulate growth hormone responsive tissues is shown. The use of a GHBP-GH therapeutic composition is shown to result in greater stimulation of GH responsive tissues with the use of less GH. Furthermore, the GHBP-GH composition is longer lasting following administration thereby permitting less frequent administration than with GH alone.

### BRIEF DESCRIPTION OF THE DRAWINGS

**Figure 1.** Schematic of Assay Procedure for detecting human growth hormone binding protein.

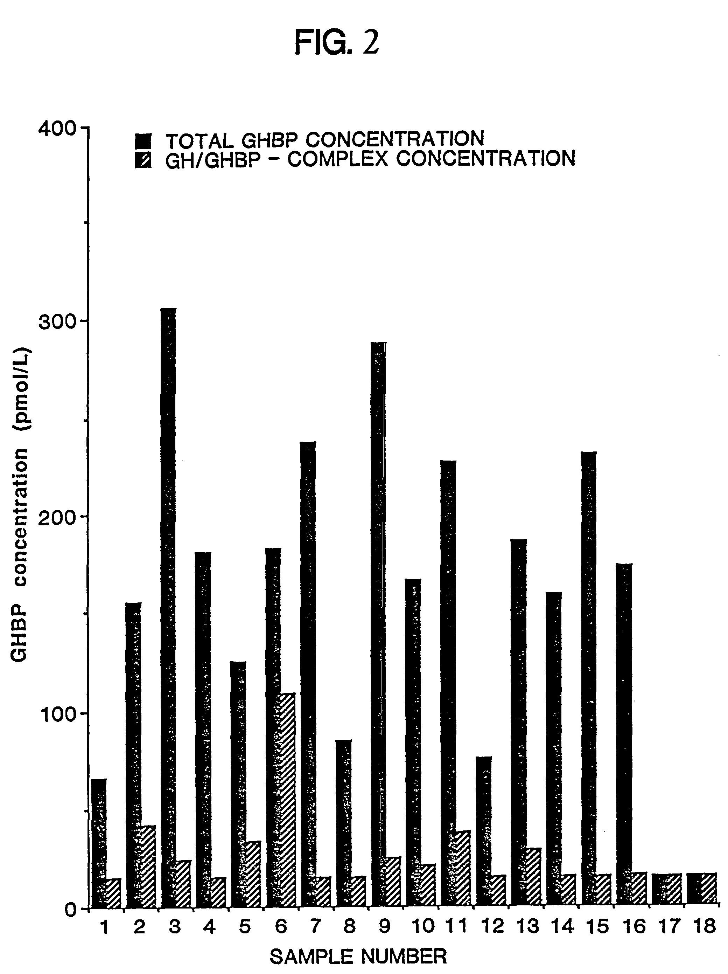

**Figure 2** Total- and hGH-bound- hGHBP levels in random serum samples from 16 healthy adults (sample #1-16) and two patients with Laron type dwarfism (sample #17 and 18).

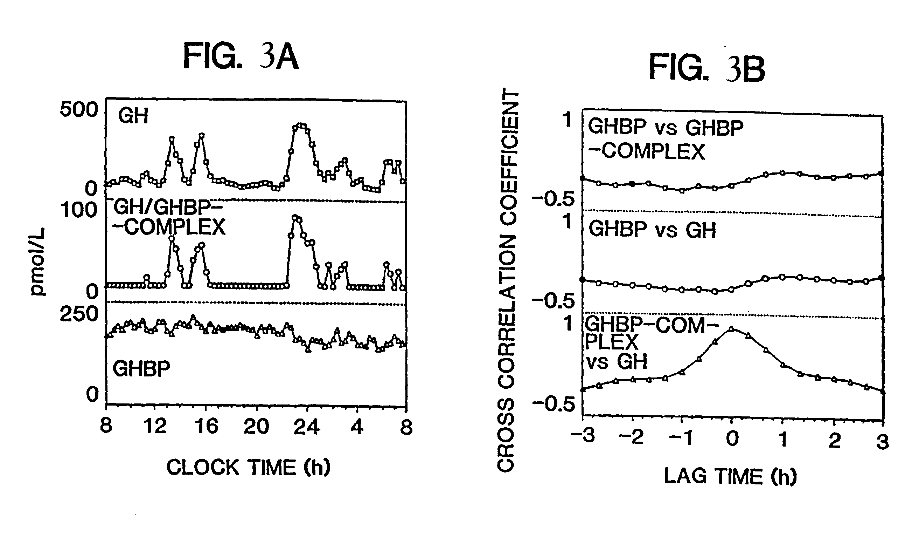

**Figure 3** Cross correlation of GH and GHBP (A) Twenty-four hour plasma profiles of GH (top panel), GH/GHBP-complex (middle panel) and total GHBP concentration (bottom panel) in samples from a a 15 year old boy (profile No 15, Table 4); (B) statistical cross correlation analysis of data from (A).
**Figure 4** GH binding protein levels in National Cooperative Growth Study (NCGS) patients indicating the log concentration of GHBP vs the age of patient. The crossbars represent mean values; solid vertical lines are plus or minus 1 SDs; dotted vertical lines are plus or minus two standard deviations (SDs). The separate black dots each represent one patient. (A) Idiopathic growth hormone deficiency (GHD) for males; (B) Idiopathic GHD for females; (C) Idiopathic short stature (ISS) for males; (D) ISS for females; (E) Turner Syndrome.

### Human Growth Hormone Binding Protein Assay

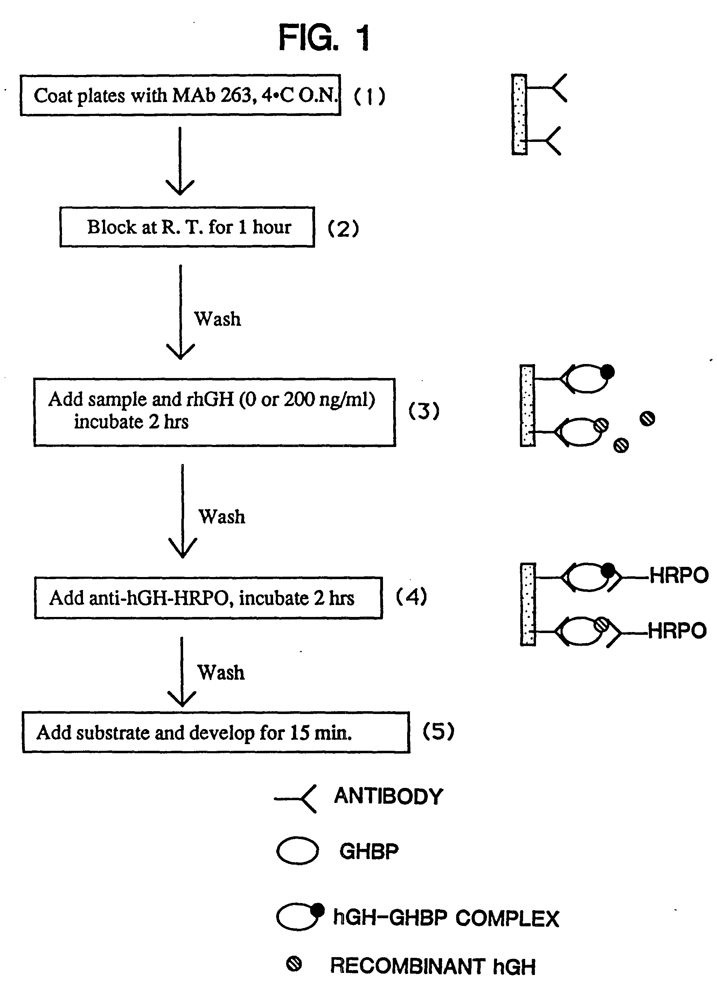

Assaying for the presence of hGHBP uses: 1) a first capture monoclonal antibody specific for the binding protein; 2) growth hormone; and 3) a second monoclonal antibody specific for growth hormone (Figure 1). The LIFA we have developed is a simple and sensitive method which allows quantitation of the total concentration of functional growth hormone binding protein in biological fluids. The assay can also be modified to measure the concentration of the circulating ligand-binding protein complex. A monoclonal antibody directed against the growth hormone-binding protein, which recognizes both free and GH bound binding protein is used to capture the binding protein on a microtiter plate (Figure 1, step 1). The samples are incubated with human growth hormone to saturate all binding sites (Figure 1, step 3) and an anti-hGH antibody is used to detect the amount of hGH (Figure 1, step 4) (endogenous and exogenous) which has bound to the binding protein. The same procedure, but without incubation with added hGH, allows quantitation of serum levels of circulating hGH-binding protein complex.

A preferred assay herein is a monoclonal antibody (MAb)-based sandwich ELISA for quantitation of GHBP in biological fluids such as human serum or plasma. The assay, which only detects functional GHBP, can be used to measure both total and complexed GHBP, and the degree of saturation of the GHBP with its ligand can therefore be calculated. In an assay developed for hGHBP, the 263 (coat) MAb binds free GHBP as well as GHBP bound to hGH, and is used to capture the GHBP on microtiter plates. The conjugated MAb MCB, which is directed against hGH, is used to detect the hGH that is bound to the immobilized GHBP. The total amount of GHBP is measured by saturating the binding sites with hGH, followed by detection of the total (endogenous and exogenous GH) bound to the GHBP. The concentration of the endogenous GH-GHBP complex is obtained by incubating the samples with the conjugated antibody without previous saturation of the GHBP with GH. The assay is sensitive and appears to cover the physiological range since random samples from 16 normal adults all had clearly detectable GHBP levels. Spike recovery experiments show that the assay is useful for measuring GHBP levels in both serum and plasma, with recoveries ranging from 89.1 to 113.6 %.

One requirement of the present assay method is for a coat antibody which recognizes the antigen both in a free and complexed form, which for hGHBP is the MAb 263.

The GH used to saturate the GHBP may be a GH analog wherein there is a binding site specific for the GHBP and one or more exposed epitopes for binding the GH specific antibody.

### Applications of the GHBP Assay

The results of the assay for GHBP and the degree of saturation of the GHBP with GH may be used for the diagnosis and treatment of growth deficiency. This assay is anticipated to provide an integrated index of the exposure of an organism to GH. It provides a more precise determination of total and free GH, which will better identify patients who are relatively GH-deficient and will benefit from GH replacement therapy. In general, the assay would be of value in any situation where GH is administered therapeutically for treating a clinical condition or where GH is present in excessive amounts and involved in causing or reflecting a clinical pathophysiology. The results provide an improved method for assessing compliance with GH replacement therapy and titrating dose and individualizing GH therapy for individual patients or those with particular clinical manifestations of GH deficiency or excess.

Use of these assay results facilitates diagnosing resistance to GH action due to decreased amounts or decreased binding activity of the GHBP or of the GH receptor, such as in hereditary or acquired syndromes. Hereditary syndromes, including Laron dwarfism and other syndromes of short stature such as ideopathic short stature are revealed when abnormalities of the GHBP are shown to exist. The assay also assists in the identification and treatment of acquired syndromes including disease states where there is diminished or excessive expression of GHBP, such as in liver disease. Other examples of the use of the present assay invention include detection of diseases of ovarian function, diseases of joints or bone, or hypothalamic or pituitary diseases causing excess GH or GHBP production. Clinical use of GHBP, or the GHBP/GH complexes as a therapeutic agent, are aided by the present assay because the measurement of GHBP or GHBP+GH after their administration assists clinical practice by allowing a determination of the presence of therapeutically effective or therapeutically ineffective amounts in bodily fluids.

All parts of the growth promoting system are interrelated and it is expected that administering growth hormone releasing factor, growth hormone inhibitory factor (somatostatin), IGF-1, or IGF-1 binding proteins, may perturb GHBP concentrations. The measurement of GHBP assists clinical practice in assessing the suitability of patients for treatment with these proteins and the efficacy of such treatments.

### Clinical Applications of GHBP Assay

Progress in our understanding of GHBP requires a quick, convenient and accurate assay for GHBP. The initial paper describing a GHBP was published in 1964 (Hadden *et al.*, Nature 202:1342 [1964]). The serum GH-binding protein was better characterized in the mouse in 1977 (Peeters, S. and Friesen, H.G., Endocrinology 101:1164 [1977]), and in 1986 two groups further characterized the existence of a human serum-binding protein for GH (Baumann, G. *et al.*, J. Clin. Endocrinol. Metab. 62:134-141 [1986]; Herington, A.C. *et al.*, J. Clin. Invest. 77:1817-1823 [1986]). When the GHBP was purified and characterized in the rabbit its amino-terminal 37 residues were shown to be identical to those of the extracellular part of the rabbit liver GH receptor (Spencer *et al.*, J. Biol. Chem. 263:7862-7867 [1988]). It has been suggested that the GHBP derives from the membrane receptor by proteolytic cleavage (Trivedi and Daughaday, Endocrinology 123:2201 [1988]) or that it is produced from a separate mRNA derived from the same gene as the full-length GH receptor (Smith *et al.*, Mol. Endo, p.984 [1989]; Baumbach *et al.,* Genes and Dev. 3:1199 [1989]). Studies of the ontogeny of the GHBP activity in man (Daughaday *et al.*, J. Clin. Endocrinol. Metab. 65:1072-74 [1987]) and the changes in the concentrations of the GHBP and hepatic GH receptors in pregnant mice (Smith and Talamantes, Endocrinology 123:1489-94 [1988]) suggest that the serum GHBP levels could be a peripheral indicator of GH receptor activity.

This idea that GHBP levels reflect GH receptor activity is supported by the finding that patients with Laron-type dwarfism, a syndrome caused by the lack of functional GH receptors, also lack GH binding activity in serum (Daughaday and Trivedi, Proc. Natl. Acad. Sci. USA 84:4636-40 [1987]; Baumann *et al.*, J. Clin. Endocrinol. Metabol 65:814-816 [1987]; Laron *et al.*, Acta Endocrinologica (Copenhagen) 121:603-608 [1989]). There are indications that in some patients with Laron type dwarfism the abnormality is caused by partial deletion of the GH receptor gene (Godowski *et al.*, Proc. Natl. Acad. Sci. USA 86:8083-8087 [1989]; Anselem *et al.*, New England J. Med. 321:989-95 [1989]), which could result in growth failure due to inability to bind GH. For this type of abnormality our assay would be particularly useful since it, in contrast to some immunoassays, would not detect the inactive protein. The serum GH binding capacity is reduced in Larondwarfism heterozygotes, and it has been suggested that measurement of the hGHBP levels in serum could be of help for genetic counseling (Laron *et al*., *supra*).

Little is known about the physiological role of the GHBP, however, recent studies have shown that the binding protein can modify the effects of growth hormone. It has been demonstrated that the GHBP alters the distribution and half-life of GH (Baumann *et al.*, Metabolism 38:330-333 [1989]), and there is also evidence to suggest that the GHBP affects the interaction of GH with its receptor on the target cells (Lim *et al.*, Endocrinology 1276:1287 [1990]). It has recently been shown that recombinant hGHBP produced in *E. coli* enhances the growth promoting effects of hGH when given to GH deficient rats, indicating that the GHBP may play an important role in the regulation of body growth in humans (see Example 5).

The LIFA is used to monitor the concentration of GHBP in the biological fluids of a patient to detect aberrant concentrations. In Examples 5, 6 and 7 numerous applications of the assay are used to monitor and evaluate the activity of human GHBP alone and complexed with hGH. These pharmacological applications include purification, dosage, frequency of administration, and duration in circulation. The LIFA is also used to monitor the activity of hGHBP from different sources, such as *E. coli*, 293 cells or from natural tissue sources.

The LIFA has application in the pharmacologic evaluation of hGHBP action in primates. The hGHBP with, and without, complexed hGH can be monitored in primates to improve the dosage and frequency of administration.

### In Primates

The ability of the GHBP to allow one to give infrequent GH injections, using similar GH doses to those used currently, yet maintain growth responses, is of clinical significance. Experiments in the monkey, using the LIFA demonstrate that clearance of a GHBP + GH complex (in one of the forms described above or in a modified form), is delayed in primates. The clearance of injected GH bound to the GHBP is slowed to a degree similar to that which we have seen in the rat. This demonstrates in primates the improved growth promoting activities of administering hGH complexed to the hGHBP.

In primates, including humans, the GHBP-complex is able to be given at infrequent intervals, greater than every 2 days, more preferably at greater than every 7 days, without a loss of efficacy compared to injections of GH alone every 1 or 2 days or daily for a week or more. In addition, the GHBP complexed with GH or alone will be given at lower total weekly doses compared to GH alone. The undesirable side effects of GH treatment, for example the diabetogenic and fluid retaining properties of GH, will be reduced with the use of the GHBP. There are other beneficial effects of using GH-GHBP complex including a heightened IGF-1 response and the ability of the GHBP to direct GH preferentially to bone. This allows the GH-GHBP complex to be used for the treatment of bone disorders, including the prevention and treatment of osteoporosis. In each situation the LIFA is used to monitor the progress of the reaction. The dosages, formulations and methods of using and making hGHBP are described in U.S. Pat. 5,057,417.

The LIFA will be used to define groups of patients who have aberrant amounts of the GHBP complex. For example, a sub-set of poorly growing children, who are relatively resistant to the growth promoting activity of GH, will be found to be deficient in the GHBP. Such children include patients with Turner's Syndrome, kidney disease, as well as a class of binding protein deficient patients who were previously described as having iodiopathic short stature. Pharmacokinetic studies delivering the GHBP or GHBP-GH complex subcutaneously, and assaying the blood levels of GHBP-GH complex using the LIFA will be performed in man to establish suitable temporal dosing regimes. Doses of GH-GHBP complex sufficient to stimulate rises in IGF-1 concentrations in blood will be determined and these doses will indicate the doses of GHBP-GH complex necessary to induce body growth. Subsequently, long-term studies in the GH-resistant children will be initiated to demonstrate the ability of the GHBP-GH complex to stimulate whole body growth, including bone growth. The LIFA will be used to monitor blood levels of the GHBP.

A primary application is to use the GHBP LIFA to monitor endogenous levels of GHBP before and during treatment for GH or GHBP deficiency. The assay of this invention serves to direct the treatment that a patient undergoes. If there is no detectable GHBP in the blood, a Laron-type syndrome may be present and IGF-1 treatment indicated. If there is a low level of GHBP in the blood, additional GH or GHBP with or without IGF-1 may be indicated. Whatever treatment regime (GH or GH + IGF-1, or GH-GHBP complex treatment) instituted the LIFA will be most valuable to determine the GHBP response to treatment. Blood GHBP concentrations will rapidly reflect the efficacy of GH treatment much more so than measurement of traditional endpoints. A lack of response of blood GHBP levels will be used as a rapid diagnostic for considering alternative strategies for treatment.

Another use of the LIFA is to detect the biological activity of endogenous blood GH. This assay uses the addition of a constant amount of GHBP followed by the addition of sample without saturating with GH. It is anticipated that patients will be detected who possess high immuno-reactive but low immunofunctional concentrations of GH. A similar assay format can be used to measure the amount of bioactive GH in any sample, especially to test batches of recombinant GH for their biological activity.

The GHBP assay can be reversed to assay for GH. The captive antibody binds GH, GHBP is complexed and the indicator antibody is specific for the bound GHBP. This permits assay of GH alone or complexed with native GHBP.

The LIFA may be used to monitor clinical administration of hGHBP, either complexed with or without hGH. A preferred use of the assay method is in a method of promoting mammalian growth and anabolism comprising: (a) determining the optimal amount of a growth hormone binding protein required to promote a growth hormone induced response; (b) measuring by LIFA the amount of growth hormone binding protein present in the biological fluids of a person suspected of being deficient in said induced response; and, (c) comparing (a) with (b), and if (a) is greater than (b), administering growth hormone binding protein in an amount sufficient to increase the level of growth hormone binding protein to said optimal amount. Another preferred clinical application of the LIFA method is to monitor a growth hormone induced response such as weight gain, bone growth, muscle growth and function, organ growth and function, skin growth, and the level of IGF-1. The organs whose growth and function are stimulated may be thymus, kidney, liver, spleen, brain heart, lungs or gonads. Yet another application of the present LIFA method is for decreasing the frequency of injecting a growth promoting amount of a growth hormone binding protein-growth hormone complex comprising: (a) determining the minimum necessary serum level of growth hormone-growth hormone binding protein complex required to maintain optimal growth; (b) measuring by LIFA the level of growth hormone binding protein present in a patient suspected of being deficient in growth hormone binding protein-growth hormone complex; and, if the complex level in (b) is less than the level in (a), then, (c) administering an amount of growth hormone binding protein-growth hormone complex sufficient to maintain the level of complex for a period greater than two days. The period may be two to fourteen days, more preferably two to eight days, and most preferably three to seven days. The optimal amount of growth hormone binding protein is defined as that equal to or greater than 90% of the average level found in healthy individuals.

### Definitions and Abbreviations:

- Capture (coat) antibody:: Antibody specific for hormone binding protein epitope such that binding of antibody does not hinder the binding of the ligand polypeptide hormone to the hormone binding protein. Antibody is attached to a solid phase and it is used to selectively bind to the binding protein and facilitate removal of the binding protein from a solution.
- Detection antibody:: Antibody which is labeled with detectable marker specific for an epitope on the polypeptide hormone and which is the detectable marker.
- HBP:: hormone binding protein; the carrier protein found in biological fluids that has affinity for a ligand polypeptide hormone and acts as a carrier for the bound ligand polypeptide hormone.
- hGH:: human growth hormone, including multiple naturally occurring forms such as 22kd, 20kd, placental variant (GHV), and variants produced by recombinant methods (Cunningham *et al.*, Science (1989) 243:1330-1336).
- GHBP:: growth hormone binding protein.
- hGHBP:: human growth hormone binding protein.

- GH:: growth hormone.
- MAb:: monoclonal antibody.
- MCB:: Monoclonal B produced by the mouse hybridoma HGH-B.
- HRPO:: horse radish peroxidase.
- BSA:: Bovine serum albumin.
- LIFA:: Ligand-mediated immunofunctional assay.

### Purification of GHBP

The purification of the GHBP used is monitored using a conventional ELISA (Fuh, G. *et al.*, J. Biol. Chem. 265, 3111-3115 [1990]). The LIFA provides a better assay for detecting the presence and assaying the amount of functional binding protein during such a purification. The LIFA ensures that functional active binding protein rather than immunologically active binding protein is purified. During hGHBP storage the LIFA is used to follow the amount of functional GHBP that remains in an active form with time. This property of the assay greatly aids in satisfying regulatory requirements concerning the stability of the GHBP during prolonged storage.

### Materials and Methods

### Selection of coat MAb

Four monoclonal antibodies to the rabbit liver growth hormone receptor, MAbs 1, 2, 5 and 7 were provided by Dr. M. Waters (University of Queensland, Queensland, Australia), and an additional two MAbs (43 and 263) were purified from mouse ascites fluid purchased from Agen, Australia. Immulon II removawell (Dynatech Laboratories, Inc., Alexandria,Va.) were coated by incubating them overnight at 4'C with antibody, 100µL per well at 5µg/mL in 50mmol/liter carbonate buffer, pH 9.6 (coating buffer). After removing the coating solution, nonspecific binding sites on the coated plates were blocked with 150µL of phosphate-buffered isotonic saline pH 7.2 (PBS) containing 5g of bovine serum albumin (BSA) per liter (blocking buffer), for In at room temperature, followed by three washes with wash buffer (0.5mL of Tween 20 and 0.1g of Thimerosal per liter of PBS). The immobilized MAbs were evaluated in three different experiments. In the first experiment, using sequential incubation steps, GHBP was first incubated with MAb coated on the well, followed by addition of radiolabelled GH (GH-I¹²⁵). In the second experiment the reaction of GHBP and GH-I¹²⁵ was carried out simultaneously in MAb coated wells. In the third experiment, GHBP was pre-incubated with hGH-I¹²⁵ overnight at 4°C and then added to the MAb coated well. In all three experiments, the nonspecific binding of tracer was determined by substituting the GHBP solution in the reaction mixture with incubation buffer (PBS containing per liter, 5g of BSA, 0.5mL of Tween® 20 and 0.1mL of Thimerosol).

### Sequential Assay

In the sequential incubation experiment, 100µL of GHBP (2.5ng/100µL) was allowed to react with immobilized MAb for 2h, washed with wash buffer, and incubated for 4h at room temperature with GH-I¹²⁵ (20,000cpm/100µL). The wells were washed 6 times in wash buffer, blotted thoroughly on adsorbent paper- and counted in a LKB series 1277 gamma counter (Pharmacia LKB Nuclear Inc., Gaithersburg, Md.) for 1 minute (Figure 6).

### Simultaneous Assay

In the simultaneous incubation experiment, 50µL of GHBP at 2.5ng/50µL in incubation buffer and 50µL of GH-I¹²⁵ at 20,000 cpm/50µL in incubation buffer were incubated in each well for 4h at room temperature. The wells were washed, blotted and counted as described above.

### Preincubation Assay

In the pre-incubation experiment, 150µL of GHBP at 2.5ng/50µL in incubation buffer and 150µL of GH-I¹²⁵ at 20,000cpm/50µL in incubation buffer were incubated in test tube overnight at 4°C. The reaction mixture was then added (100µL per well) to the MAb coated well and incubated for 4h at room temperature. The wells were washed, blotted and counted as previously described.

### Enzyme-conjugated Anti-hGH Antibodies

The anti-hGH detection MAb was selected for conjugation because it did not give detectable displacement of the GHBP and contains no overlapping determinants with the GHBP (Cunningham *et al*., Science 243:1330-1336 [1989]). The antibodies were purified from ascites fluid using protein A-Sepharose® (Repligen Corp., Cambridge MA.) following established procedures (Ey *et al.*, Immunochem. 15:429 [1978]; Goding, J.W., J. Immunol. Meth. 20:241 [1978]) and stored sterile in 0.01M sodium phosphate, 0.15M sodium chloride, pH 7.2 (PBS) at 4°C. Purified MAbs were conjugated to horseradish peroxidase (Nakane and Kawaoi, J. Histochem. Cytochem. 22:1084 [1974]) and stored at -20°C in 50% glycerol.

### LIFA assay standards

The recombinant human growth hormone binding protein (GHBP) was purified from a mammalian cell line following the procedure outlined by Spencer *et al.* (J. Biol. Chem. 263:7862-7867 [1988]). The purified GHBP amino acid composition as determined by quantitative amino acid analysis matched what was theoretically expected for the cloned gene product. The purified GHBP was homogeneous based on analysis of SDS-gel electrophoresis. The concentration of GHBP in the purified preparation was established by Scatchard analysis (Scatchard, G., Annals of the New York Academy of Sciences 51:660-672 [1949]), and dilutions of this sample in PBS containing per liter, 5g of BSA, 5.0mM EDTA, 0.5ml of Tween 20 and 0.1g of Thimerosal (assay buffer) were then used as standards in the LIFA. GHBP produced in *E. coli* was also used, but surprisingly found to give non-parallel dilution curves in the assay. Therefore, the preferred GHBP is either natural GHBP or GHBP produce by cells which produce GHBP in a native configuration, that is, glycosylated.

### Recombinant human GH

Recombinant human growth hormone (GH) was supplied by Genentech Inc., South San Francisco, California, USA.

### Serum and plasma samples

Serum and plasma (EDTA, citrate and heparin as anticoagulants) samples were obtained from healthy adult volunteers (9 men and 7 women, 26 to 43 years old). The samples were centrifuged and stored at -70°C until assayed.

### Therapeutic Treatment Following GHBP Evaluation

For the various purposes of this invention, the COMPOSITIONS (GHBP+GH, GH, IGF-I, IGF-1+binding protein) administered to the mammal or avian by any suitable technique, including parenterally, and can be administered locally or systemically.

The COMPOSITIONS are directly administered to the mammal by any suitable technique, including parenterally, intranasally, or orally. The specific route of administration will depend, e.g., on the medical history of the patient, including any perceived or anticipated side or reduced anabolic effects using hGH or IGF-I alone, and the growth defect to be corrected. Examples of parenteral administration include subcutaneous, intramuscular, intravenous, intraarterial, and intraperitoneal administration. Most preferably, the administration is by continuous infusion (using, e.g., minipumps such as osmotic pumps), or by injection using, e.g., intravenous or subcutaneous means. Preferably, the COMPOSITIONS administration is subcutaneous. The administration may also be as a single bolus or by slow-release depot formulation. Most preferably, the IGF-I or IGF-1 plus binding protein is administered continuously by infusion, most preferably subcutaneously; GHBP + GH or GH alone is administered daily subcutaneously by injection. Most preferably, the GHBP + GH is administered intermittently every 2 or more days, weekly, biweekly, or monthly.

The IGF-I is suitably administered together with its binding protein, for example, BP53, which is described in WO 89/09268 published October 5, 1989 and by Martin and Baxter, J. Biol. Chem., 261: 8754-8760 (1986). This protein is an acid-stable component of about 53 Kd on a non-reducing SDS-PAGE gel of a 125-150 Kd glycoprotein complex found in human plasma that carries most of the endogenous IGFs and is also regulated by GH. The IGF-I is also suitably coupled to a receptor or antibody or antibody fragment for administration. Similarly, the GH can be delivered coupled to another agent such as an antibody, an antibody fragment, or one of its binding proteins.

The COMPOSITIONS to be used in the therapy will be formulated and dosed in a fashion consistent with good medical practice, taking into account the clinical condition of the individual patient (especially the side effects of treatment with hGH or IGF-I alone or growth retardation after continuous GH treatment), the site of delivery of the COMPOSITIONS, the method of administration, the scheduling of administration, and other factors known to practitioners. The "effective amounts" of each component for purposes herein are thus determined by such considerations and must be amounts that enhance the anbolic growth of the treated patient.

As a general proposition, the total pharmaceutically effective amount of each of the COMPOSITIONS administered parenterally per dose will be in the range of about 1 µg/kg/day to 50 mg/kg/day of patient body weight, although, as noted above, this will be subject to a great deal of therapeutic discretion. More preferably, this dose is at least 2 µg/kg/day, and most preferably at least 5 µg/kg/day for each hormone. If given continuously, the IGF-I, IGF-1 + binding protein, GHBP + GH and GH are each typically administered at a dose rate of about 1 µg/kg/hour to about 100 µg/kg/hour, either by 1-4 injections per day or by continuous subcutaneous infusions, for example, using a minipump. An intravenous bag solution may also be employed. The key factor in selecting an appropriate dose is the anabolic result obtained, as measured by increases in body weight gain, lean body mass, or statutory growth approximating the normal range, or by other criteria for measuring anabolic activity as defined herein as are deemed appropriate by the practitioner.

The COMPOSITIONS are also suitably administered by sustained-release systems. Suitable examples of sustained-release compositions include semipermeable polymer matrices in the form of shaped articles, e.g., films, or microcapsules. Sustained-release matrices include polylactides (U.S. Pat. 3,773,919, EP 58,481), copolymers of L-glutamic acid and gamma-ethyl-L-glutamate (U. Sidman *et al.,* Biopolymers, 22, 547-556 (1983)), poly(2-hydroxyethyl methacrylate) (R. Langer *et al.*, J. Biomed. Mater. Res., 15: 167-277 (1981), and R. Langer, Chem. Tech., 12: 98-105 (1982)), ethylene vinyl acetate (R. Langer *et al.,* Id.) or poly-D- (-)-3-hydroxybutyric acid (EP 133,988). Sustained-release compositions also include liposomally entrapped COMPOSITIONS. Liposomes containing COMPOSITIONS are prepared by methods known per se: DE 3,218,121; Epstein *et al.*, Proc. Natl. Acad. Sci. U.S.A., 82: 3688-3692 (1985); Hwang *et al.,* Proc. Natl. Acad. Sci. U.S.A., 77: 4030-4034 (1980); EP 52,322; EP 36,676; EP 88,046; EP 143,949; EP 142,641; Japanese Pat. Appln. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal COMPOSITIONS therapy.

For parenteral administration, in one embodiment, the IGF-I and GH are formulated generally by mixing each at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the COMPOSITIONS each uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine,; monosaccharides, disaccharides, and other carbohydrates including cellulose or its derivatives, glucose, mannose, or dextrins; chelating agents such as EDTA; sugar alcohols such as mannitol or sorbitol; counterions such as sodium, and/or nonionic surfactants such as polysorbates, poloxamers, or PEG.

The COMPOSITIONS are each typically formulated individually in such vehicles at a concentration of about 0.1 mg/ml to 100 mg/ml, preferably 1-10 mg/ml, at a pH of about 4.5 to 8. Full-length IGF- I is generally stable at a pH of no more than about 6; des(1-3)- IGF-I is stable at about 3.2 to 5; hGH and GHBP are stable at a higher pH of, e.g., 6.0-7.8. It will be understood that use of certain of the foregoing excipients, carriers, or stabilizers will result in the formation of IGF-I or GH salts.

In addition, the COMPOSITIONS preferably the full-length IGF- I, are suitably formulated together in a suitable carrier vehicle to form a pharmaceutical composition that does not contain cells. In one embodiment, the buffer used for formulation will depend on whether the composition will be employed immediately upon mixing or stored for later use. If employed immediately after mixing, theCOMPOSITIONS can be formulated in mannitol, glycine, and phosphate, pH 7.4. If this mixture is to be stored, it is formulated in a buffer at a pH of about 6, such as citrate, with a surfactant that increases the solubility of the GH at this pH, such as 0.1% polysorbate 20 or poloxamer 188. The final preparation may be a stable liquid or lyophilized solid.

The COMPOSITIONS to be used for therapeutic administration must be sterile. Sterility is readily accomplished by filtration through sterile filtration membranes (e.g., 0.2 micron membranes). Therapeutic COMPOSITIONS (IGF-I, IGF-1 + binding protein, GHBP + GH and GH compositions) generally are placed into a container having a sterile access port, for example, an intravenous solution bag or vial having a stopper pierceable by a hypodermic injection needle.

COMPOSITIONS ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous GH solution, and the resulting mixture is lyophilized. The infusion solution is prepared by reconstituting the lyophilized GH using bacteriostatic Water-for-Injection.

GHBP plus GH ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous IGF-I solution, and the resulting mixture is lyophilized. The infusion solution is prepared by reconstituting the lyophilized GHBP plus GH using bacteriostatic Water-for-Injection. The GHBP+GH formulation may further contain mannitol, glycine, a buffer, and a non-ionic surfactant. The formulation of the subject invention may optionally include one of several types of non-ionic surfactants, such as the polysorbates (e.g. polysorbate 20, 80, etc.) and the poloxamers (e.g. poloxamer 188). When polysorbate 80 is used the molar ratio of GHBP+GH:polysorbate 80 is 1:0.07-30, advantageously 1:0.1-10, and most advantageously 1:3. On a weight to volume basis, polysorbate 80 is added in amounts of about 0.001 to about 2% (w/v), in order to enhance further the stability of the hGH. Polysorbate 80, in concentrations above 0.01% (w/v) reduces the amount of aggregation forming upon lyophilization. In addition to improved shell life, the surfactant containing formulation of the subject invention inhibits the formation of protein aggregates when the reconstituted formulation is shaken.

When GHBP+GH is administered, it must contain one or more of its binding proteins. A well characterized such binding protein is the high-affinity growth hormone binding protein (GHBP) constituting the extracellular domain of the GH receptor that circulates in blood and functions as a GHBP in several species [Ymer and Herington, Mol. Cell. Endocrino., 41:153 (1985); Smith and Talamantes, Endocrinology, 123: 1489-1494 (1988); Emtner and Roos, Acta Endocrinologica (Copenh.), 122: 296-302 (1990)], including man (Baumann *et al*., J. Clin. Endocrinol. Metab., 62: 134-141 (1986); EP 366,710 published 9 May 1990; Herington *et al.*, J. Clin. Invest., 77: 1817-1823 (1986); Leung *et al*., Nature, 330: 537-543 (1987)). A second BP with lower affinity for GH has also been described that appears to be structurally unrelated to the GH receptor (Baumann and Shaw, J. Clin. Endocrinol. Metab., 70: 680-686 (1990)).

Novel formulations of zinc, GHBP and GH result in a stable composition suitable for prolonged storage, and for therapeutic administration. Therapeutic formulations containing the Zn²⁺ ion are stable allowing therapeutic administration of the formulation. The formulation aspect of the present invention is thus directed to such formulations, and to all associated formulations and as a means for effectively stabilizing GHBP+GH. The formulation contains zinc, and substantially pure GHBP and GH free of contaminating proteins or infectious agents found in humans. Formulations of the present invention may additionally contain a pharmaceutically acceptable buffer, amino acid, bulking agent and/or non-ionic surfactant. These include, for example, buffers, chelating agents, antioxidants, preservatives, cosolvents, and the like; specific examples of these could include, trimethylamaine salts ("Tris buffer"), and disodium edetate.

### hGH Compositions

As used herein, the terms "human growth hormone" or "hGH" denote human growth hormone produced, for example, from natural source extraction and purification, and by recombinant cell culture systems. The native sequence of hGH and its characteristics are set forth, for example, in Hormone Drugs, Gueriguigan *et* al., U.S.P. Convention, Rockville, MD (1982) The terms likewise cover biologically active human growth hormone equivalents; e.g., differing in one or more amino acid(s) in the overall sequence. Further, the terms as used in this application are intended to cover substitution, deletion and insertion amino acid variants of hGH, or post translational modificationsExamples of such variants are described in PCT Pub. WO90/04788 published 3 May 1990. The hGH used in the formulations of the present invention is generally produced by recombinant means as previously discussed. The formulation of recombinant GHBP+GH is substantially pure, free of other human proteins, free of infectious agents such as the human immunodeficiency virus (HIV) and it is soluble. "Substantially pure" GHBP+GH means GHBP and GH that is free of proteins with which it ordinarily is associated in bodily fluids such as blood, plasma and serum. Ordinarily, substantially pure means GHBP and GH which is greater than about 95% pure by weight of total protein, and preferably greater than 98% pure by weight.

### Formulation Amino acids

In an alternative formulation embodiment, a pharmaceutically acceptable amino acid, for example glycine, is added to the GHBP and GH:zinc ion formulation. When glycine is present, the molar ratio of GHBP+GH:glycine is 1:5-600 In addition to glycine, amino acids such as alanine, glutamine, asparagine, arginine or lysine or derivatives of such amino acids may be used in the subject formulation. Such amino acids are particularly advantageous when lyophilizing the formulation to create a sufficient mass to form a stable, dry caked formulation.

### Non-Ionic Surfactant

In another embodiment a non-ionic surfactant is added to the GHBP+GH formulation. The formulation of the subject invention may optionally include one of several types of non-ionic surfactants, such as the polysorbates (e.G. polysorbate 20, 80 etc.) and the poloxamers (e.g. poloxamer 188). Advantageously polysorbate 80 is used, and the molar ratio of hGH:polysorbate 80 may be 1:0.03-60. On a weight to volume basis, polysorbate 80 is added in amounts of about 0.001 to about 2% (w/v), in order to enhance further the stability of the GHBP and GH. Polysorbate 80, in concentrations above 0.01% (w/v) may reduce the amount of inactive aggregates forming upon lyophilization and reconstitution. The use of non-ionic surfactants improves formulation stability when exposed to shear and surface stresses without causing denaturing of the protein. Further, such surfactant containing GHBP and GH formulations, may be employed in aerosol devices such as those used in a pulmonary dosing, and needleless jet injector guns. Such delivery formulations may be improved by the addition of non-ionic surfactants in the range of 0.1-5% (w/v).

As used herein, the expression mammal refers to any mammal but especially primates, bovine, ovine, canine, feline, equine and rodentia. Specifically it includes human, cows, horses, rats, mice, rabbits, monkeys, cats, dogs and pigs. The term avain refers to any bird, particularly chicken, turkey, duck and goose.

The foregoing written specification is considered to be sufficient to enable one skilled in the art to practice the invention. The present invention is not to be limited in scope by the constructs deposited, since the deposited embodiment is intended to illustrate only certain aspects of the invention and any constructs that are functionally equivalent are within the scope of this invention. The deposit of material herein does not constitute an admission that the written description herein contained is inadequate to enable the practice of any aspect of the Invention, including the best made thereof, nor is it to be construed as limiting the scope of the claims to the specific illustrations that they represent. Indeed, various modifications of the invention In addition to those shown and described herein will become apparent to those skilled in the art from the foregoing description and fall within the scope of the appended claims. The following examples are intended to illustrate the best mode now known for practicing the invention, but the invention is not to be considered limited to these examples.

### EXAMPLE 1

### LIFA ASSAY PROCEDURE FOR GHBP

The LIFA assay procedure developed for measuring the GHBP is as follows. Ninety-six-well microtiter plates (Corning Glass Works, Corning, New York) were coated with MAb 263 by incubating overnight at 4°C with 100µl/well of antibody at 20µg/ml in 50mmol/liter of sodium carbonate buffer, pH 9.6 (coat buffer) (see Figure 1, step 1). After removal of the coating solution, the coated plates were blocked with 150µl per well of 5g/liter of BSA in PBS for 1h at room temperature (Figure 1, step 2), and washed six times with 0.5g/liter of Tween 20 in PBS (wash buffer).

Standards diluted in assay buffer or samples (50µM serum or plasma and 50µM assay buffer) were dispensed onto the coated wells (100µl/well) (Figure 1, step 3). Plates were sealed and incubated at room temperature for 2h with gentle agitation. Plates were washed six times with wash buffer. Recombinant hGH at 200ng/ml or assay buffer was then added (100µl/well) and incubated at room temperature for 2h. Plates were washed six times with wash buffer before addition of horseradish peroxidase (HRPO) labelled MAb MCB (100µl/well) Figure 1, step 4). After further incubation for 2h at room temperature, the plates were washed six times with wash buffer. Freshly prepared substrate solution (0.4g of o-phenylenediamine dihydrochloride in one liter of PBS plus 0.4 ml of 30% hydrogen peroxide) was added to the plates (100µl per well) and incubation carried out in the dark for 15 min at room temperature Figure 1, step 5. The reaction was stopped by the addition of 100µl of 2.25mol/L sulfuric acid and absorbance at 490nm determined on a Vmax plate reader (Molecular Devices, Menlo Park, CA). A standard curve was generated by plotting absorbance vs. log of GHBP concentration, using a 4-parameter nonlinear regression curve litting program. Sample concentrations were obtained by interpolation of their absorbance on the standard curve.

### EXAMPLE 2

### DETERMINING GHBP IN BIOLOGICAL FLUIDS

### Application of the LIFA method

Total and GH-bound GHBP levels in random serum samples from 16 healthy adults and two patients with Laron type dwarfism are shown in Figure 6. GH-BP levels were detectable in samples from all normal subjects (Figure 6; patients 1-16). In contrast, GHBP concentrations were undetectable (< 30 pmol/L) in both patients with Laron-type dwarfism (Figure 6: patients #17, #18).

### EXAMPLE 3

### MONITORING OF GHBP IN GROWTH PROMOTION

The LIFA of the present invention may be used to monitor the concentration of GHBP in the biological fluids of a patient, and If the level is inadequate for the desired rate of growth, additional GHBP administered. An example of low GHBP levels is in Laron dwarfism; whereas a high GHBP levels may be present in patients with excess GH secretion. If the level GHBP is insufficient for the desired rate of growth, additional GHBP alone or complexed to hGH may be administered. The optimal level of GHBP may be determined by the methods discussed above, in combination with measuring the level of GHBP in a series of normal healthy individuals. GHBP may be evaluated in any mammalian system, preferably in rodents and primates.

### In Rodents

Two rodent models of GH deficiency are used: 1) rats where the gland producing GH, the pituitary, is surgically removed (hypophy-sectomized rats) and 2) animals genetically deficient in growth hormone (dwarf rats, Charlton, H.W. *et al.*, J. Endo. 119:51-58 [1988]). These rats are treated with human GH (hGH) alone or hGH coupled to human GHBP which is produced recombinantly in *E. coli* or alternatively in mammalian 293 cells. Several indices of growth are measured to assess the effect of the binding protein on GH-induced body growth. Monitoring of the level of GHBP and GH is required to determine the metabolic fate of administered GHBP and complexed GH.

### Hypophysectomized Rats

Recombinant hGHBP and hGH are given either alone or in combination to hypophysectomized rats, a recognized model for measuring GH bioactivity (Thorngren, K-G. & Hansson L.I. Acta. Endo. 75:653-668 [1977]). Human GH (0.03, 0.1 and 0.3 mg/kg, as 7 daily injections) induces a dose-related weight gain while injections of *E. coli*-derived hGHBP at these same 3 doses produces no effect by itself. However, co-administration of 0.3mg/kg hGHBP with 0.1mg/kg hGH not only gives greater weight gain than 0.1mg/kg hGH alone (p<0.01), but also induces greater weight gain than three times more hGH (22.0 ± 3.6 vs 17.1 ± 2.1g respectively; mean ± s.d., p<0.01). Longitudinal bone growth parallels body weight gain. Thus, co-administration of 0.3mg/kg hGHBP and 0.1mg/kg hGH gives greater bone growth than 0.3mg/kg hGH (102 ± 14 vs 84 ± 17 microns/day; p<0.05), and 0.1mg/kg hGHBP plus hGH gives greater bone growth than hGH alone (99 ± 6 vs 72 ± 17 microns/day; p<0.01).

The liver, spleen and kidney are all significantly larger following co-adminstration of hGHBP (0.3mg/kg) with hGH (0.1mg/kg) than with 0.1mg/kg hGH alone: liver (5.5 ± 0.4 vs 4.6 ± 0.6g; p<0.01), spleen (292 ± 46 vs 240 ± 34mg; p<0.05), and kidney (836 ± 60 vs 716 ± 57mg; p<0.05). The weights of liver, spleen and kidney in excipient treated rats are 4.5 ± 0.2g, 193 ± 32mg, and 687 ± 58mg, respectively. These responses to hGH are at least doubled by hGHBP. The serum concentrations of IGF-1 and hGH 24h after the last injection are markedly elevated by co-administration of the two highest doses of hGHBP with hGH, while hGHBP causes as much as 20-fold more hGH to be present after 24h.

An ELISA (Fuh, G. *et al.*, J. Biol. Chem. 265:3111-3115 [1990]) for hGHBP adapted for use in serum shows the reason for the persistence of the GH in the blood 24 hours after the seventh subcutaneous bolus injection was given to the rats. The hGHBP is only detectable in the animals co-administered hGHBP and hGH. When the GHBP is given alone it disappears from the blood more rapidly than when the GHBP is given complexed to GH. These findings from measuring the GHBP in blood suggest that it is the persistence of the GH + GHBP complex in the blood of the rats that causes many or all of the above improved activities of GH. The LIFA method of the present invention is therefore used to follow GHBP during its preparation, storage, use and in body fluids following GHBP administration.

### Dwarf Rat System

We also compared hGH and hGHBP in a dwarf rat which has a pituitary GH content 5-10% of normal, grows slowly, and responds to GH treatment (Charlton, H.W. *et al.*, J. Endo. 119: 51-58 [1988]) Co-administration of 0.27mg/kg of hGHBP with 0.27mg/kg hGH increases weight gain compared to 0.27mg/kg hGH alone (11.1± 4.2 vs 7.5 ± 1.7g; p<0.05). Co-administration of all three doses of hGHBP with 0.27mg/kg of hGH significantly increasing bone growth compared to 0.27mg/kg hGH alone (low 33.5 ± 5.8, medium 38.6 ± 8.6, high 35.5 ± 5.0, vs 26.0 ± 4.1 microns/day; p<0.05). Serum IGF-1 concentrations are elevated by co-administration even compared to 0.81mg/kg hGH alone (high hGHBP 136 ± 45 vs 90 ± 16ng per ml; p<0.05) as were hGH concentrations (high hGHBP, 609 ± 240 vs 73 ± 22 pg per ml; p<0.0001).

### GHBP From Mammalian 293 Cells

In marked contrast, hGHBP produced in human 293 cells completely inhibits GH responses in hypophysectomized rats. Weight gains after 10 daily s.c. injections of hGH alone are 11.3 ± 2.5, 16.4 ± 2.1 and 21.1 ± 2.1g at 0.03, 0.1, and 0.3mg/kg/day respectively. When hGH at 0.03 and 0.1mg/kg/day is co-administered with a 2-fold molar excess of 293-derived hGHBP this weight gain is abolished (3.0 ± 2.1 and 3.0 ± 1.6g, respectively) compared to the hGH and excipient (2.3 ± 1.6g) groups. This difference between the growth responses induced using these 2 forms of hGHBP may be due to a difference in hGH clearance from the circulation which is reduced about 10 fold for GHBP derived from *E. coli* (Moore, J.A. *et al*, Proc. US Endocr. Soc. 71, Abstract #1652 [1989]).or purified from natural sources (Baumann, G. & Shaw, M.A., J. Clin Endocrinol. Metab., 70:680-686 [1990]; Baumann, G., Shaw, M.A. & Buchanan, T.A., Metabolism. 38:330-333[1989].

The clearance (ml/min/kg) of hGH in normal male rats, following an i.v. bolus of hGH alone is 18.6 ± 3.4, for hGH co-administered with GHBP from rabbit sera is 2.1 ± 0.2, for GHBP from *E. coli* 1.9 ± 0.4, or from 293 cells 41.3 ± 16.7. Therefore, for the 293-derived hGHBP the clearance of hGH is increased two fold, suggesting a correlation between *in vivo* potency and hGH clearance. The decreased clearance of hGH complexed to the *E. coli* -derived hGHBP may be due to the complex being of sufficient size (Mr>40 kDa) to escape filtration by the kidney. Proteins produced in 293 cells can have heterogenous carbohydrate patterns, possibly due to incomplete glycosylation, causing them to be rapidly cleared by the liver, which may explain the rapid clearance of 293-derived hGHBP.

In this instance the 293-derived GHBP therefore acts as an inhibitor of GH action, compared to the enhancing activities of the *E. coli*-derived protein. This difference in activity appears to be due to the differing clearances of the two molecules from the blood. The present invention aids in similarly discriminating inhibitory and stimulatory binding proteins on the basis of their clearance from the blood.

### Persistence of GHBP

This series of experiments shows the value of the knowledge gained from a GHBP assay, and naturally follows from the above rat experiments. On the basis of the prolonged half-life of the *E. coli*-derived GH plus GHBP complex in blood, the GHBP allows GH to persist in the blood for sufficient time to allow less frequent GH injections when the GH is coupled to GHBP.

In 2 separate studies we inject hGH by itself, or combined and co-injected with hGHBP, in GH deficient dwarf rats. In the first study hGH or hGH plus hGHBP are given daily or every 2 or 4 days for 8 days. The second study is designed so that hGH or hGH plus hGHBP are given daily or every 3 or 6 days for 18 days. In both studies hGHBP gives greater growth responses than hGH alone no matter the injection interval. These studies show that when hGH is injected with hGHBP the injection frequency can be greatly reduced, to once or twice a week, without reducing the size of the growth response.

Female dwarf rats (Study A, 12-15 weeks of age, 110-130g; Study B, 50 -70 days of age, 95-110g) are randomized into groups of 8 (Study A) or 7 (Study B), and injected i.p. with tetracycline as an intravital marker of bone growth. All injections of GH or GHBP are given subcutaneously in a volume of 100 microlitres of solution. In each study, all rats are given a daily injection of either excipient or test compounds and weighed daily.

The hGH used is rhGH (Genentech Lot N9267AX, G042A) dissolved In sterile water. The GHBP used is produced in *E. coli* and purified. In this case the GHBP has an altered sequence to the GHBP used above, the molecule being produced by removing the exon 3 coding domain giving a 1-5, 27-238, peptide sequence.

In the first experiment the hGH and hGHBP are prepared to be injected in a volume of 0.1ml as:
a) hGH 0.25mg/ml or
b) hGH 1mg/ml
c) hGH 1mg/ml) + hGHBP (2mg/ml)

The 8 regimes of injection are:
1) Daily injections of a), b), and c),
2) Injections every 2 days of b) and c)
3) Injections every 4 days of b) and c)
4) Injections of excipient every day.

Therefore in this design the animals injected s.c. every 2nd day receive only half the GH dose of the animals given daily injections and the rats injected every 4th day receive only a quarter of the cumulative GH dose. In the second experiment the hGH is prepared as:
a) 0.33mg/ml,
b) 1mg/ml, or
c) 2mg/ml.

The hGH + hGHBP solutions are 0.33, 1 or 2mg/ml of hGH combined with 2-fold more hGHBP (0.66, 2 or 4mg/ml, respectively). The 9 regimes all injected in 0.1ml s.c. are:
1) Excipient control
2) Daily hGH injections 33 micrograms/day
3) Daily hGH injections 100 micrograms/day
4) Daily hGH injections 33 micrograms/day + 66µg of GHBP
5) Daily hGH injections 100 micrograms/day + 200µg of GHBP
6) Every 3rd day hGH injections 100 micrograms/shot
7) Every 6th day hGH injections 200 micrograms/shot
8) Every 3rd day injections 100 micrograms hGH + 200µg GHBP/shot
9) Every 6th day injections 200 micrograms hGH + 400µg GHBP/shot
Therefore, in this design the animals injected s.c. every 3rd or 6th day receive the same cumulative GH dose (0.33mg/kg/day) as those injected s.c. with the low dose of GH.

The response to hGH is increased at all frequencies of injection by combining hGHBP with the hGH. It is surprising that in study A despite decreasing the injection frequency from daily to every 2 days, and thereby reducing the cumulative hGH dose by half, the weight gain response GHBP + GH injections is the same. For hGH injections the weight gain response is markedly reduced when the injections are given every 2 or 4 days. The weight gain in response to eight daily injections of 0.25mg/kg hGH is identical to that for only 2 injections of the same cumulative dose of hGH given every 4 days.

In the second Experiment, two doses of hGH are given daily with or without a 2-fold excess of hGHBP. The response to hGH is greatly increased by combining hGHBP with the hGH (see below). (In a subsequent study in the dwarf rat the maximal weight gain response to hGH was increased when the hGH was complex with and injected daily s.c. with hGHBP.) As in the previous experiments GHBP improved the IGF-1 response to the GH. This appears to be due to the GHBP-GH complex causing a preferential and disproportionate growth of the liver, an activity lacking when GH is delivered alone. Giving the same total dose of hGH at 3 or 6 day intervals gives a poor growth response (compared to hGH given daily, Group 2). The weight gain response to combined treatment with GH + GHBP is much greater. The effect of daily hGH alone is directly compared with hGH + hGHBP given every 3 or 6 days. Infrequent injections of the hGHBP-hGH combination are as effective as daily injections of hGH. These data clearly show that the co-administration of hGH + hGHBP allows the growth response to the hGH to be maintained with infrequent injection regimes. Co-administration of GH + GHBP allows the interval between injections to be extended to 6 days (weekly) without a loss of activity on longitudinal bone growth (measured by the tetracycline labelling technique) compared to injections of the same dose of GH injected daily. The co-administration of GH + GHBP also allows a smaller dose of GH to be given less frequently for an equivalent growth response (injections every 2 or 3 days at 1/2 to 1/3 the dose in the rat).

| **STUDY B: Bone Growth and Weight Gain in 18 days** | | | | |
|---|---|---|---|---|
| Group | Bone Growth (microns) &SD | | Weight Gain (g) &SD | |
| 1) Excipient | 34.0 | 8.0 | 10.3 | 3.6 |
| 2) low GH | 53.8 | 8.3 | 27.8 | 3.3 |
| 3) Hi GH | 62.1 | 8.4 | 34.0 | 5.4 |
| 4) Low GH + GHBP | 73.4 | 9.7 | 28.1 | 4.9 |
| 5) Hi GH + GHBP | 95.1 | 6.5 | 47.0 | 7.4 |
| 6) GH / 3 days | 37.7 | 10.3 | 15.2 | 3.7 |
| 7) GH / 6 days | 36.5 | 11.7 | 16.8 | 6.2 |
| 8) GH + GHBP / 3 days | 56.9 | 15.8 | 28.0 | 6.4 |
| 9) GH + GHBP / 6 days | 47.0 | 4.6 | 18.5 | 4.8 |

### In Primates

In normal juvenile Rhesus monkeys GHBP was monitored after administration of GHBP + hGH or after hGH alone. The somatogenic and anabolic response was determined by measuring IGF-1 concentrations. The monkeys received either hGH daily or hGHBP+ hGH weekly, and there was an excipient injected control group. The results, primarily from blood IGF-1 concentrations, showed that GHBP enhances the biologically activity of GH in primates. The dose of hGH (administered with twice the molar ratio of hGHBP) was 0.35 mg/kg injected weekly, the doses of hGH injected daily were 0.05mg/kg or 0.35mg/kg. Serum analyses indicated that the maximum IGF-1 response to hGH + hGHBP is greater than for hGH alone at either dosage. As measured, the hGH serum life when administered in combination with hGHBP was increased 2.2 times over that of hGH administered alone. The administration of 0.35mg/kg hGH daily stimulated serum IGF-1 less than a single weekly administration of hGH (0.35mg/kg) complexed with hGHBP at a 2:1 molar ratio. Weekly administration of hGH+hGHBP (1:2 molar ratio, given subcutaneously as a bolus), resulted in a physiological response greater than daily hGH administration of the same hGH dose, or a seven-fold greater total dose. Therefore, lesser amounts of hGH can be administered and less frequent injections given if hGH is complexed with hGHBP. In summary, in the rat and in the monkey, GHBP enhances GH activity, so that in humans a similar enchancement is expected.

The mechanism of the above effects of the GHBP on body growth could be explained by the greatly delayed absorption of the GH-GHBP complex from s.c. injections and then the delayed clearance from the blood of the GH-GHBP complex. Both these effects are demonstrated. The magnitude of this effect is quite surprising as the absorption of free GHBP was similar to that for GH alone. These pharmacokinetic mechanisms explain a large part of the increased activity, and the ability to give less frequent injections of the GHBP + GH complex. These discoveries are surprising as there is no prior art showing that increasing the half-life of GH in the blood would inevitably lead to an increased activity. If a molecule is retained in the blood its access to tissues will be limited, yet degradation of the molecule in the blood will continue. If the GH is bound to the GHBP access of the GH to a cellular GH receptor would be expected to be modified. It is clear that for the molecule to be active on tissues it must pass from the bloodstream into the tissues, so that there are limits to the degree of delayed clearance that is desirable.

### EXAMPLE 4

### Analysis of 24-hour Plasma Profiles of GHBP, GH/GH-GHBP-complex and GH In Healthy Children

We have used the LIFA to measure GHBP levels in plasma profiles from healthy children. GH was measured by IRMA. Fifteen 24h plasma profiles from 12 healthy children (3 girls and 9 boys) of different ages (6-17 years), heights (-3.7 to +3.5 SDS) and pubertal stages (1 to 4) were examined. Blood was withdrawn continuously for 24h and collected in 20 min fractions. Time series for GH, GHBP and GH/GHBP-complex were analyzed by cross-correlation and Fourier analysis. GH was secreted in a pulsatile fashion in all subjects. The concentration of the GH/GHBP-complex varied during the sampling period, and the changes correlated significantly with the GH pulses with correlation coefficients reaching maximum at zero time lag. In contrast, the changes in the total GHBP concentration were minor (CV∼10%), and not correlated to GH pulses. Fourier analysis showed similar spectral power patterns for GH and GH/GHBP-complex, suggesting a diurnal rhythm (12-24h periods) as well as components of higher frequencies (around 4 h periods). In spite of the subtle fluctuations in the total GHBP concentration, Fourier transformation revealed a marked diurnal rhythm, while components of higher frequencies were much less abundant. We conclude that the variations in total GHBP during a 24h sampling period are small and that the levels can be estimated from a single random blood sample.

### Materials and Methods

### Subjects

Twelve children, 3 girls and 9 boys, of different ages (6-15 years old), heights (-3.7 to +3.5 SDS) and pubertal stages (stage 1-4), were investigated at the Children's Hospital, Göteborg, Sweden. Two of the subjects were studied on more than one occasion (Table 4). Height at the time of the study was expressed in SD scores compared to normal Swedish children (Karlberg P, Taranger J, Engström I, Lichtenstein H, Svennberg-Redegren I. The somatic development of children in a Swedish urban community. Acta Pediatrica Scand. 1976; Suppl. 258:1). All children were healthy and well nourished, and had normal thyroid, liver and kidney function. Coeliac disease was excluded. Children with classical GH deficiency were not included in the study. The testicular volume was measured by orchidometer, and the pubertal stages were classified according to Tanner (Tanner JM, Whitehouse RH. Clinical longitudinal standards for height velocity, weight velocity and stages of puberty. (Arch. Dis. Child. 51:170-179[1976]).

**TABLE 1**

| **SUBJECT CHARACTERISTICS** | | | | | | | |
|---|---|---|---|---|---|---|---|
| Profile No | Subject | Sex | Age | Height (SDS) | Weight (SDS) | Puberty | |
| | | | | | | B/T^{a} | PH^{b} |
| 1 | A | Female | 6 0/12 | +2.5 | +2.0 | 1 | 1 |
| 2 | B | Female | 12 8/12 | -1.9 | -2.0 | 1 | 1 |
| 3 | C | Female | 13 3/12 | -1.0 | 0 | 3 | 2 |
| 4 | D | Male | 7 11/12 | -0.3 | +0.5 | 1 ml | 1 |
| 5 | E | Male | 11 0/12 | -2.3 | -2.0 | 2 ml | 1 |
| 6 | F | Male | 11 2/12 | -2.0 | -1.5 | 3 ml | 1 |
| 7 | G | Male | 11 3/12 | +3.0 | +2 | 2 ml | 1 |
| 8 | H | Male | 13 0/12 | +1.0 | +1.0 | 15 ml | 3 |
| 9 | H | Male | 13 9/12 | +0.8 | +0.8 | 15 ml | 4 |
| 10 | I | Male | 12 5/12 | +0.7 | +0.1 | 4 ml | 1 |
| 11 | I | Male | 12 6/12 | +0.7 | 0 | 4 ml | 1 |
| 12 | I | Male | 13 3/12 | +0.4 | -0.4 | 5 ml | 1 |
| 13 | J | Male | 14 0/12 | -1.8 | -1.8 | 6 ml | 1 |
| 14 | K | Male | 14 7/12 | -2.5 | -1.5 | 12 ml | 3 |
| 15 | L | Male | 14 8/12 | -3.7 | -3.2 | 5 ml | 2 |

| | | | | | | | |
|---|---|---|---|---|---|---|---|
| ^{a} Breast development (B), testicular volume (T). | | | | | | | |
| ^{b} Pubic hair (PH). | | | | | | | |

### Study protocol

The Children stayed at the hospital for at least 2 days. They were given a normal diet, with breakfast at 08.00h, lunch at 12.00h, dinner at 17.00h, and were allowed normal activity and sleep. A heparinized needle (Carmeda, Stockholm, Sweden) was inserted on the first evening. The following morning, at 08.00h-09.00h, blood withdrawal began through a thrombogenic catheter (Carmeda) inserted through the needle and connected to a constant withdrawal pump (Swemed AB, Göteborg, Sweden). The rate of withdrawal was 0.5-2 ml/h and the volume of the tubing was 0.1-0.2 ml. The heparinized reservoir tubes were changed every 20 min for 24h, thus giving 72 samples. The blood samples were kept at room temperature and centrifuged within 24h. After centrifugation the plasma was frozen until assayed.

### GH measurements

Plasma GH concentration was determined in duplicate using a polyclonal antibody-based IRMA (Pharmacia, Sweden) and the WHO First International Reference Preparation hGH 66217 as standard. Intra-assay variation was 3.2 to 3.5 % at GH levels between 2-100 mU/L. Interassay variation was 5.0 % and 2.7 % at GH concentrations of 10 mU/L and 40 mU/L, respectively. When appropriate, a conversion factor of 2.7U/mg, and a molecular weight of 22 000 was used to express GH concentrations in pmol/L.

### GHBP measurements

Total GHBP was measured by LIFA as previously discussed in detail . Briefly, ninety-six-well microtiter plates (Corning Glass Works, Corning, New York) were coated with a monoclonal antibody directed against GHBP (MAb 263, Agen, Australia) by incubating overnight at 4°C with 100 µL/well of antibody at 10 µg/mL in coat buffer. The coated wells were blocked and washed. Standards (recombinant hGHBP, Genentech Inc.) or samples (50 µL per well) were dispensed into the coated wells containing 50 µl/ well of 200 ng/ml rhGH. Plates were sealed, incubated at room temperature for 2 h with gentle agitation, then washed before addition of a monoclonal anti-hGH antibody (MAb MCB, Genentech Inc) conjugated to horseradish peroxidase (100 µl/well). After further incubation for 2 h at room temperature, the plates were washed six times with wash buffer. Freshly prepared substrate solution (0.4 g of *o*-phenylenediamine dihydrochloride in one liter of PBS plus 0.4 mL of 30% hydrogen peroxide) was added to the plates (100 µl per well) and the incubation carried out in the dark for 15 min at room temperature. The reaction was stopped by the addition of 100 µl of 2.25 mol/L sulfuric acid and the absorbance at 490 nm determined. The same procedure, without the addition of rhGH to the samples, was used to measure the plasma concentration of the GH/GHBP-complex. The detection range in the LIFA was 15.6 to 1000 pmol/L. The intra- and interassay coefficients of variation were 7.3 % and 11.3 %, respectively.

### Statistical analysis

The rhythmicity of the 24h profiles was analyzed by Fourier transformation. The original GH, GHBP and GH/GHBP-complex concentration time series were smoothed with a 3-point moving average (weights w₋₁=w₊₁=1/4, w₀=1/2) in order to reduce the influence of high frequency components. The smoothed series were analyzed as Fourier expansions(Chatfield C. The analysis of time series. Chapman and Hall, London. 1989). The results are expressed as a power spectrum, where the amplitude is plotted as a function of frequency. To analyze the data for correlations between GH, GHBP and GH/GHBP-complex, cross-correlation followed by Box-Jenkins autoregressive modeling (Haugh L, Box GEP. Identification of dynamic regression (distributed lag) models connecting two time series. Journal of the American Statistics Association. 1977; 72:121-130) was used.

### Results

### Plasma profiles of GH GH/GHBP-complex and total GHBP:

Twenty-four hour plasma profiles of GH, GH/GHBP-complex and total GHBP from one representative subject (profile # 15) are shown in Fig. 7. Plasma concentrations of both GH (top panel) and GH/GHBP-complex (middle panel) varied over a wide range during the sampling period, and the changes appeared to be synchronized in time. In contrast, the total concentration of GHBP (lower panel) was much less variable, and did not seem to be influenced by the changes in GH and GH/GHBP-complex concentration.

### Plasma concentrations of GH, GHBP and GH/GHBP-complex

Plasma concentrations of GH, GHBP and GH/GHBP-complex are shown in Table 5. The values given are the mean and coefficients of variation (CV) for each individual 24h profile as well as the group average. The mean concentration of GHBP, GH/GHBP-complex and GH varied among different individuals (range 109-247pmol/L, 13-109 pmol/L and 32-215 pmol/L for GHBP, GH/GHBP-complex and GH, respectively). The highly pulsatile nature of GH and GH/GHBP-complex plasma profiles were reflected in their high CV (156.0% and 47.2%, respectively). The percentage of GH that was bound to GHBP in high GH peaks (250 pmol/L) was 14.6%. The concentration of total GHBP was much less variable during the sampling period, as illustrated by the low coefficient of variation (9.6 %) (Table 5). The complete 24h profiles of total GHBP concentration from all subjects illustrated that levels vary among individuals and that the concentration for each subject is relatively constant throughout the day.

**Table 2**

| **Mean plasma concentration of total GHBP, GH/GHBP-complex and GH.** | | | | | | |
|---|---|---|---|---|---|---|
| Profile No | Total Mean (pmol/L) | GHBP CV (%) | GH/GHBP Mean (pmol/L) | -complex CV (%) | GH Mean (pmol/L) | P CV (%) |
| 1 | 109.3 | 9.4 | 34.8 | 25.6 | 73.1 | 129.6 |
| 2 | 235.2 | 9.5 | 12.9 | 113.9 | 83.3 | 178.5 |
| 3 | 179.0 | 12.7 | 26.2 | 111.0 | 99.8 | 128.9 |
| 4 | 148.5 | 8.1 | 24.1 | 25.8 | 40.4 | 107.7 |
| 5 | 198.9 | 7.8 | 47.3 | 51.0 | 167.9 | 176.7 |
| 6 | 160.1 | 8.6 | 25.7 | 28.6 | 36.6 | 160.7 |
| 7 | 215.0 | 5.5 | 39.8 | 22.5 | 31.6 | 151.0 |
| 8 | 247.4 | 7.7 | 108.8 | 15.3 | 215.2 | 121.6 |
| 9 | 217.5 | 9.3 | 80.1 | 17.2 | 189.1 | 133.7 |
| 10 | 126.2 | 11.8 | 28.2 | 46.4 | 163.4 | 151.3 |
| 11 | 120.4 | 13.8 | 20.7 | 37.0 | 101.9 | 125.7 |
| 12 | 168.7 | 9.2 | 34.3 | 43.4 | 99.8 | 119.4 |
| 13 | 130.0 | 11.1 | 29.5 | 41.5 | 140.9 | 154.9 |
| 14 | 191.1 | 11.8 | 19.8 | 107.5 | 103.7 | 99.1 |
| 15 | 199.8 | 7.0 | 45.4 | 21.9 | 39.3 | 153.5 |
| Mean: | 176.5 | 9.6±0.6 | 38.5 | 47.2±9.2 | 110.0 | 139±6.3 |

### Discussion

The objective in monitoring GHBP was to investigate the possible diurnal variations in the plasma concentration of GHBP in healthy children, and to determine if fluctuations in GHBP concentrations were correlated with the episodic release of GH. The subjects included in our study differed in age, sex, pubertal stage, height and GH levels. From a practical point of view we believe that a most useful discovery from the data is that total GHBP concentration shows only minor variations during a 24h sampling period, implying that a single blood sample should give a good estimation of the total GHBP level. Nevertheless, rigorous analysis of the data revealed that the small (CV~10 %) fluctuations in total GHBP plasma concentration account for a significant circadian rhythm. No significant cross-correlation between GH pulses and changes in total GHBP concentration was found. In contrast, the plasma concentration of GH/GHBP-complex showed rapid fluctuations, which were highly correlated with the changes in GH concentration. Fourier analysis showed that the plasma patterns of both GH and GH/GHBP-complex follow a diurnal rhythm but also possess components of higher frequencies (around 4h periods).

The described LIFA, which was used for the GHBP measurements has the advantages that only functional GHBP is detected and that endogenous GH, which fluctuates rapidly over a wide range, does not affect the measurement of total GHBP concentrations. The assay can also measure the concentration of the GH/GHBP-complex. It was recently reported that when the ratio of GHBP to GH exceeds 1:1, trimers can form consisting of one GH molecule and two GHBP molecules. The trimer can not be bound by the capture antibody, so the GH/GHBP-complex that Is measured in the LIFA is the heterodimer formed by one GH molecule and one GHBP molecule. When GH concentration increases,(e.g. when rhGH Is added to the sample in the LIFA or during endogenous GH peaks), the trimer [GH-(GHBP)2] dissociates and dimers [GH-GHBP] are formed, which can be bound by the MAb 263. This implies that all the GHBP, including GHBP molecules in endogenously formed trimers, are detectable in the assay for the total GHBP concentration.

We found that about 15 % of GH in high peaks (>250 pmol/L) appear to be bound in the GH/GHBP-complex, but the total bound fraction of GH may be higher since some GH may have formed trimers with the GHBP.

The variation in the plasma concentration of GHBP in serial samples has previously been addressed in two studies (Snow, KJ, Shaw MA, Winer LM, Baumann G. Diurnal pattern of plasma growth hormone-binding protein in man. J Clin Endocrinol Metab. ;70:417-420 (1990); Hochberg Z, Amit T, Zadik Z. Twenty-four-hour profile of plasma growth hormone-binding protein. J. Clin Endocrinol Metab. 72:236-239 (1991)). The study by Snow *et al.*, which was carried out in adults with low GH levels, agrees with our results that there is no major variation in total GHBP levels during the sampling period. However, since GH pulses were absent or very low in the study by Snow *et al.*, the possibility that there could be GH induced variation in GHBP levels in subjects with pulsatile GH secretion could not be excluded. In another study, by Hochberg *et al.*, GH and GHBP levels were measured in samples obtained from normal children with pulsatile GH secretion and it was concluded that within 30 min the majority of the GH pulses were accompanied by GHBP pulses. This is in contrast to the present results, where only minor changes in the total GHBP concentration were detected and they were not correlated with the GH pulses. The reason for the discrepancy between the two studies is not clear, but may be due to differences between the GHBP assays. The LIFA directly measures total GHBP (i.e. the sum of free GHBP and GH-bound GHBP), while the assay used by Hochberg *et al*. is based on the binding of radiolabeled hGH to the GHBP and the values are then corrected for interference by endogenous GH. Since our data regarding the GH/GHBP-complex indicate that the complex is formed and cleared rapidly, it is possible that the apparent GHBP pulses, which Hochberg *et al* observed 30 min after a GH pulse may reflect the desaturation of the GHBP, thereby allowing more labeled GH to be bound.

The total GHBP levels varied over a wide range in different subjects (109-247 pmol/L) and it is probably that these levels are correlated to differences in age, sex, pubertal stage, growth velocity, etc. We conclude that GHBP levels are relatively constant throughout the day and a single or pooled blood sample should be sufficient to estimate total GHBP concentration. This finding should facilitate comparisons with larger populations and illustrates the value of GHBP measurement as a diagnostic tool.

### EXAMPLE 5

### GHBP DETERMINATIONS OF NORMAL AND SHORT STATURE CHILDREN

Growth hormone binding protein was assayed using the LIFA assay for human GHBP. The results are described in Tables 6, 7, 8 and 9 below. These tables contain summary statistics on GHBP levels in normal children (Table 3) as well as in children with short stature due to three different etiologies: idiopathic growth hormone deficiency (GHD) (Table 4), (ISS) (Table 5) and Turner syndrome (Table 9). Normal data were obtained from samples in Genentech's control and from collaborations with outside investigators. The samples from children with short stature were obtained as part of an ongoing post-marketing surveillance project from Protropin® human growth hormone, the Genentech National Cooperative Growth Study (NCGS). Now ISS children are now longer ideopathic in that the GHBP deficiency likely reflects an underlying growth hormone receptior deficiency.

The summary statistics for normal children are presented by sex and age and represent our best estimate of a normal range for GHBP. These statistics consist of the mean and mean plus or minus 2 standard deviations (SD) and were determined from the logged (base 10) values of the GHBP levels, then converted back to the original units. Sample sizes used In the estimates are included with the summary statistics. Note that there were sufficient data to perform these calculations for male and female children aged, 3, 4 and 6 through 15 only. Data from children of other ages and from adults were too sparse to allow a good estimate of the mean.

The summary statistics listed for each of the etiologies of short stature are the sample size, mean and mean plus or minus one SD. These values were computed in the same manner as that described for the normals. The statistics are printed by age and sex for all available data regardless of sample size. The units of GHBP are in pmole/liter.

**TABLE 3**

| **Growth Hormone Binding Protein Norms (GHBP Normal Range)** | | | | | |
|---|---|---|---|---|---|
| Sex | Age | N | Mean -2 SD | Mean | Mean +2 SD |
| Male | 3 | 20 | 57.4 | 127.3 | 282.5 |
| Male | 4 | 21 | 64.6 | 120.2 | 223.5 |
| Male | 6 | 31 | 56.5 | 111.6 | 220.6 |
| Male | 7 | 31 | 78.2 | 143.0 | 261.7 |
| Male | 8 | 34 | 62.7 | 178.5 | 507.7 |
| Male | 9 | 36 | 64.9 | 198.1 | 604.7 |
| Male | 10 | 37 | 62.5 | 226.9 | 822.8 |
| Male | 11 | 40 | 70.7 | 234.6 | 779.0 |
| Male | 12 | 48 | 79 4 | 238.0 | 713.3 |
| Male | 13 | 33 | 72.5 | 231.7 | 739.9 |
| Male | 14 | 37 | 67.6 | 97.7 | 578.4 |
| Male | 15 | 33 | 51.7 | 173.4 | 581.8 |
| Female | 3 | 15 | 77.4 | 149.3 | 288.0 |
| Female | 4 | 17 | 62.0 | 179.3 | 518.6 |
| Female | 6 | 33 | 57.8 | 142.7 | 351.9 |
| Female | 7 | 32 | 73.5 | 175.2 | 417.7 |
| Female | 8 | 33 | 93.7 | 230.9 | 568.8 |
| Female | 9 | 36 | 90.8 | 215.7 | 512.4 |
| Female | 10 | 32 | 71.2 | 244.6 | 841.0 |
| Female | 11 | 33 | 97.5 | 285.8 | 838.3 |
| Female | 12 | 36 | 87.7 | 228.7 | 596.8 |
| Female | 13 | 36 | 113.0 | 305.9 | 827.8 |
| Female | 14 | 35 | 99.7 | 260.2 | 678.7 |
| Female | 15 | 28 | 122.4 | 345.8 | 976.5 |

**TABLE 4**

| **Growth Hormone Binding Protein Norms (Idiopathic GHD: GHBP Levels)** | | | | | |
|---|---|---|---|---|---|
| Sex | Age | N | Mean -1 SD | Mean | Mean +1 SD |
| Male | 1 | 1 | - | 73.5 | - |
| Male | 3 | 3 | 40.3 | 63.7 | 100.7 |
| Male | 4 | 5 | 56.3 | 95.4 | 161.7 |
| Male | 5 | 3 | 79.5 | 96.5 | 117.1 |
| Male | 6 | 7 | 76.9 | 97.3 | 123.1 |
| Male | 7 | 1 | - | 59.4 | - |
| Male | 8 | 4 | 87.3 | 127.8 | 187.1 |
| Male | 9 | 5 | 81.9 | 128.9 | 203.0 |
| Male | 10 | 12 | 73.5 | 150.3 | 307.2 |
| Male | 11 | 9 | 108.5 | 177.5 | 290.2 |
| Male | 12 | 14 | 106.8 | 193.5 | 350.4 |
| Male | 13 | 16 | 102.1 | 164.4 | 264.6 |
| Male | 14 | 17 | 83.0 | 149.8 | 270.4 |
| Male | 15 | 17 | 122.4 | 191.1 | 298.3 |
| Male | 16 | 5 | 132.1 | 231.4 | 405.3 |
| Male | 17 | 4 | 136.0 | 165.0 | 200.2 |
| Male | 18 | 1 | - | 772.5 | - |
| Female | 3 | 1 | - | 55.8 | - |
| Female | 5 | 2 | 77.4 | 82.5 | 87.9 |
| Female | 6 | 3 | 117.8 | 157.6 | 210.8 |
| Female | 8 | 2 | 200.3 | 241.3 | 290.7 |
| Female | 9 | 2 | 43.8 | 59.7 | 81.3 |
| Female | 10 | 3 | 83.4 | 253.9 | 772.7 |
| Female | 1 | 10 | 91.0 | 162.5 | 290.3 |
| Female | 2 | 7 | 89.0 | 189.9 | 405.2 |
| Female | 13 | 2 | 86.6 | 154.5 | 275.9 |
| Female | 5 | 2 | 294.0 | 398.6 | 540.4 |

**TABLE 5**

| **Growth Hormone Binding Protein Norms (Turner Syndrome: GHBP Levels)** | | | | | |
|---|---|---|---|---|---|
| Sex | Age | N | Mean -1 SD | Mean | Mean +1 SD |
| Female | 4 | 2 | 93.2 | 118.1 | 149.5 |
| Female | 5 | 4 | 84.1 | 108.3 | 139.4 |
| Female | 6 | 7 | 65.3 | 135.8 | 282.2 |
| Female | 7 | 4 | 97.5 | 146.1 | 218.9 |
| Female | 8 | 5 | 116.3 | 194.5 | 325.4 |
| Female | 9 | 10 | 107.9 | 199.0 | 367.2 |
| Female | 10 | 11 | 105.8 | 182.7 | 315.7 |
| Female | 11 | 8 | 181.0 | 241.9 | 323.2 |
| Female | 12 | 8 | 139.4 | 286.4 | 588.2 |
| Female | 3 | 9 | 133.3 | 241.8 | 438.7 |
| Female | 14 | 15 | 222.2 | 321.4 | 464.8 |
| Female | 15 | 6 | 101.0 | 189.0 | 353.5 |
| Female | 16 | 6 | 147.4 | 237.6 | 383.0 |
| Female | 17 | 2 | 117.6 | 136.0 | 157.4 |

**TABLE 6**

| Growth Hormone Binding Protein Norms (Idiopathic Short Stature: GHBP Levels) | | | | | |
|---|---|---|---|---|---|
| Sex | Age | N | Mean -1 SD | Mean | Mean +1 SD |
| Male | 2 | 1 | - | 37.3 | - |
| Male | 3 | 8 | 46.0 | 100.2 | 218.5 |
| Male | 4 | 1 4 | 68.6 | 96.3 | 135.4 |
| Male | 5 | 2 4 | 61.0 | 86.1 | 121.6 |
| Male | 6 | 1 7 | 47.9 | 65.1 | 88.4 |
| Male | 7 | 3 5 | 52.9 | 83.0 | 130.0 |
| Male | 8 | 2 9 | 61.1 | 90.8 | 135.0 |
| Male | 9 | 2 5 | 64.7 | 110.7 | 189.3 |
| Male | 1 0 | 4 5 | 69.5 | 108.3 | 168.6 |
| Male | 1 1 | 4 3 | 67.0 | 106.1 | 168.0 |
| Male | 1 2 | 7 7 | 70.5 | 109.5 | 170.2 |
| Male | 1 3 | 7 3 | 73.5 | 117.9 | 189.3 |
| Male | 1 4 | 7 1 | 70.8 | 110.4 | 172.0 |
| Male | 1 5 | 5 1 | 64.7 | 113.8 | 200.3 |
| Male | 1 6 | 1 2 | 55.9 | 102.9 | 189.4 |
| Male | 1 7 | 5 | 80.3 | 112.7 | 158.2 |
| Male | 1 8 | 2 | 64.5 | 195.0 | 589.6 |
| Male | 19 | 1 | - | 93.8 | - |
| Male | 2 1 | 1 | - | 184.3 | - |
| Male | 2 3 | 1 | - | 36.9 | - |
| Female | 1 | 1 | - | 63.3 | - |
| Female | 2 | 2 | 56.8 | 57.4 | 58.0 |
| Female | 3 | 2 | 45.3 | 88.2 | 171.4 |
| Female | 4 | 2 | 87.1 | 88.0 | 89.0 |
| Female | 5 | 4 | 50.8 | 91.4 | 164.5 |
| Female | 6 | 5 | 45.6 | 84.7 | 157.4 |
| Female | 7 | 6 | 76.6 | 109.2 | 155.6 |
| Female | 8 | 9 | 75.4 | 116.0 | 178.6 |
| Female | 9 | 9 | 89.8 | 120.7 | 162.2 |
| Female | 1 0 | 1 9 | 102.1 | 174.9 | 299.7 |
| Female | 1 1 | 3 1 | 84.3 | 139.3 | 230.1 |
| Female | 1 2 | 2 0 | 89.4 | 150.1 | 252.2 |
| Female | 1 3 | 1 7 | 110.1 | 146.3 | 194.3 |
| Female | 1 4 | 9 | 102.1 | 160.9 | 253.7 |
| Female | 1 5 | 4 | 56.5 | 110.7 | 216.8 |
| Female | 1 6 | 2 | 148.1 | 182.2 | 224.1 |

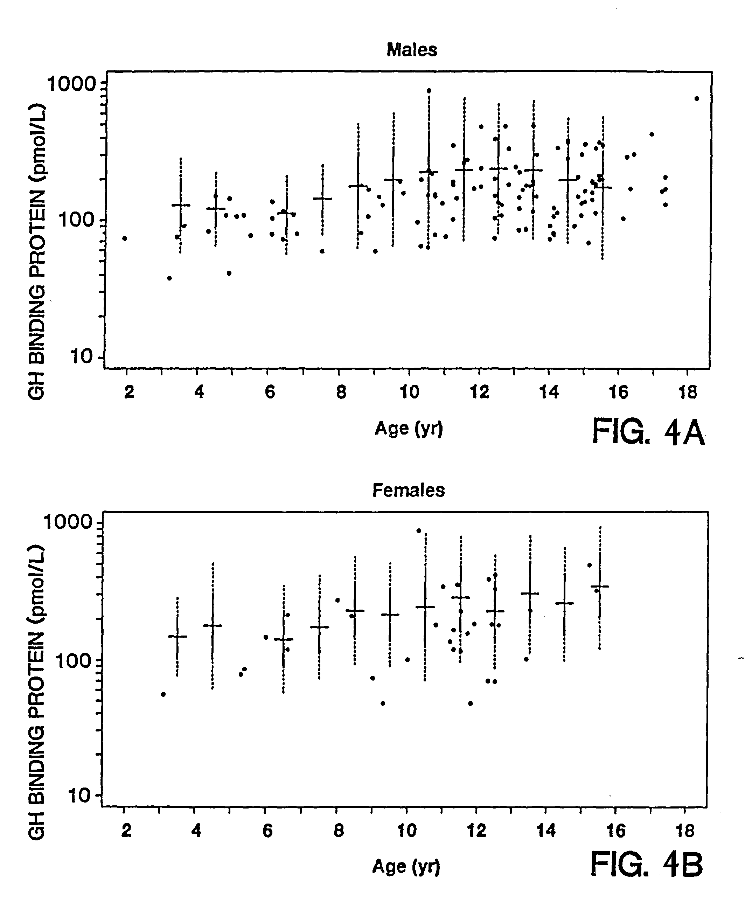

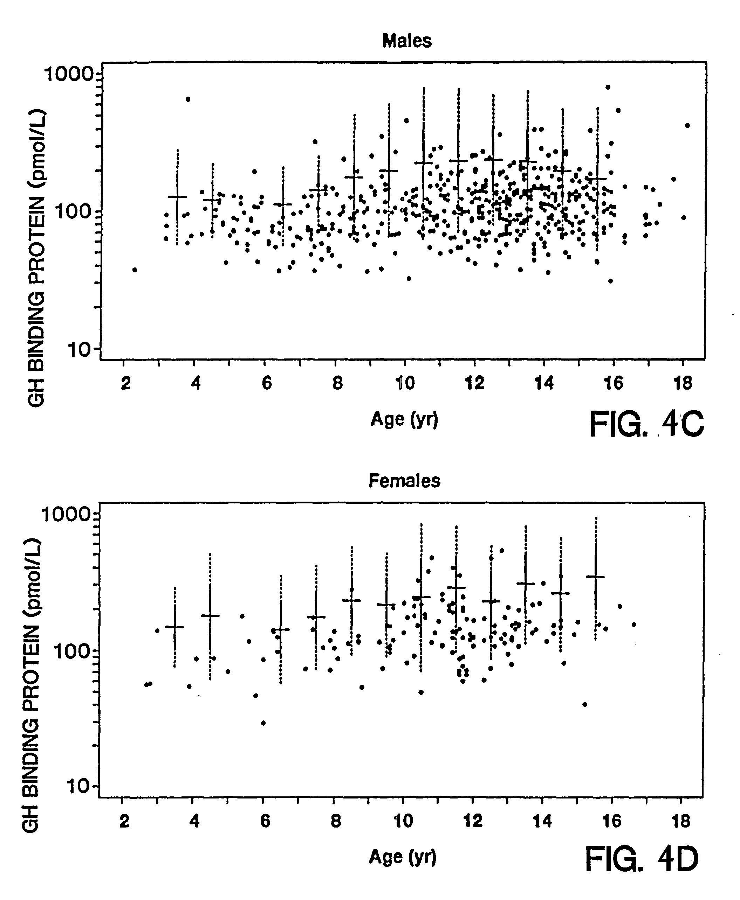

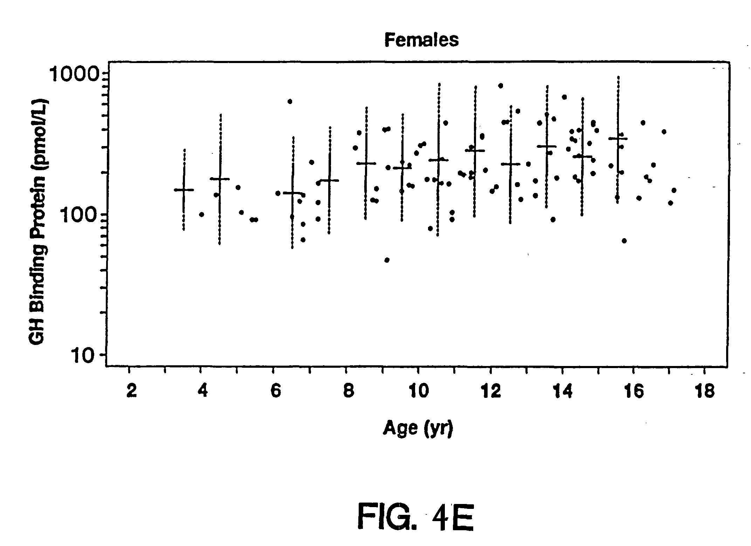

Figure 4 graphically illustrates the difference between normal GHBP and various etiologies. Plotted are GH binding protein levels from the National Cooperative Growth Study patients (log concentration of GHBP vs the age of patient). The crossbars represent mean values; solid vertical lines are plus or minus 1 SDs; dotted vertical lines are plus or minus two SDs. The separate black dots each represent one patient. (A) Idiopathic GHD for males; (B) Idiopathic GHD for females; (C) Idiopathic short stature for males; (D) Idiopathic short stature for females; (E) Turner Syndrome.

The clinical utility of the LIFA assay for distinguishing between normals and various etiologies can be seen in Figure 4(A-E). This is particularly pronounced in figure 4 C and D where the idiopathic short stature patients are predominantly below the mean for normal in both males and females. This provides a clinically valuable diagnostic test for evaluating the level of GHBP, and indirectly a presumptive determination of growth hormone receptor.

Ideopathic short stature is no longer ideopathic in that it can now be viewed as a state of relative GH resistance. Based upon the relative lack of GHBP this resistance is likely due to a deficiency in GH receptors that are capable of responding to GH. Current therapy of ISS involves daily hGH Injections. One approach to the deficiency in GH receptors is to administer higher doses of GH to stimulate those receptors that are present. A dosage of from 1.1 to 10 times that now used is expected to increase the GH response. Alternatively, now that the underlying basis of ISS is revealed, new treatment options different from that currently used are suggested. In GH deficiency and Turner's syndrome, situations of normal GHBP and presumably normal GH receptor responsiveness, GHBP + GH is the logical treatment. In ISS patients, GHBP +GH may also be used to maximize the response by those GH receptors present. However, in ISS, IGF-1 therapeutic treatment is also indicated. IGF-1 is given alone or with IGF binding protein it may be coadministered with GH, and/or with GHBP. Since ISS patients have reduced GHBP, the GHBP + GH combination elevates the response by those GH receptors present. Administration of therapeutic amounts of IGF-1 or IGF-1 plus IGF BP elevates the effective serum IGF-1, thus partially circumventing the defective GH response and stimulating IGF-1 dependent responses.

## Claims (Claims for the following Contracting State(s): AT, BE, CH, LI, DE, DK, FR, GB, IT, LU, NL, SE)

1. A formulation comprising a therapeutically effective amount of growth hormone binding protein and growth hormone, for use in a method of stimulating a mammal's or avian's growth hormone responsive tissues.

2. The formulation of claim 1 wherein the dose of growth hormone and growth hormone binding protein is from about 1 µg/kg/day to about 50 mg/kg/day.

3. The formulation of claim 1 or claim 2 further containing one or more ingredient selected from the following: non-ionic surfactant, zinc ion and glycine.

4. The formulation of claim 3 further comprising a therapeutically effective amount of insulin-like growth factor-1.

5. The formulation of claim 4 further comprising a therapeutically effective amount of insulin-like growth factor binding protein.

6. Use of growth hormone binding protein and growth hormone in the manufacture of a therapeutically effective formulation for stimulating a mammal's or avian's growth hormone responsive tissues.

7. A formulation comprising growth hormone binding protein, growth hormone and a non-ionic surfactant.

8. A formulation comprising growth hormone binding protein, growth hormone and zinc.

9. The formulation of claim 8 further comprising a slow release formulation.

## Claims (Claims for the following Contracting State(s): ES, GR)

1. A formulation comprising a therapeutically effective amount of growth hormone binding protein and growth hormone, for use in a method of stimulating a mammal's or avian's growth hormone responsive tissues.

2. The formulation of claim 1 wherein the dose of growth hormone and growth hormone binding protein is from about 1 µg/kg/day to about 50 mg/kg/day.

3. The formulation of claim 1 or claim 2 further containing one or more ingredient selected from the following: non-ionic surfactant, zinc ion and glycine.

4. The formulation of claim 3 further comprising a therapeutically effective amount of insulin-like growth factor-1.

5. The formulation of claim 4 further comprising a therapeutically effective amount of insulin-like growth factor binding protein.

6. Use of growth hormone binding protein and growth hormone in the manufacture of a therapeutically effective formulation for stimulating a mammal's or avian's growth hormone responsive tissues.

7. A formulation comprising growth hormone binding protein, growth hormone and a non-ionic surfactant.

8. A formulation comprising growth hormone binding protein, growth hormone and zinc.

9. The formulation of claim 8 further comprising a slow release formulation.

10. A process for the manufacture of a composition for use in a method of stimulating mammal's or avian's growth hormone responsive tissues,the process comprising formulating a therapeutically effective amount of growth hormone binding protein and growth hormone.

11. The process of claim 10 wherein the dose of growth hormone and growth hormone binding protein is from about 1 µg/kg/day to about 50 mg/kg/day.

12. The process of claim 10 or claim 11 wherein the composition further contains one or more ingredient selected from the following: non-ionic surfactant, zinc ion and glycine.

13. The process of claim 12 wherein the composition further comprises a therapeutically effective amount of insulin-like growth factor-1.

14. The process of claim 13 wherein the composition further comprises a therapeutically effective amount of insulin-like growth factor binding protein.

15. A process for the manufacture of a composition comprising formulating growth hormone binding protein, growth hormone and a non-ionic surfactant.

16. A process for the manufacture of a composition comprising formulating growth hormone binding protein, growth hormone and zinc.

17. The process of claim 16 wherein the composition further comprises a 5 slow release formulation.

## Patentansprüche (Patentansprüche für folgende(n) Vertragsstaat(en): AT, BE, CH, LI, DE, DK, FR, GB, IT, LU, NL, SE)

1. Formulierung, die eine therapeutisch wirksame Menge an Wachstumshormon-Bindungsprotein und Wachstumshormon umfasst, zur Verwendung in einem Verfahren zur Stimulierung von auf Wachstumshormon ansprechendem Gewebe eines Säugetiers oder Vogels.

2. Formulierung nach Anspruch 1, worin die Dosis an Wachstumshormon und Wachstumshormon-Bindungsprotein etwa 1 µg/kg/Tag bis etwa 50 mg/kg/Tag beträgt.

3. Formulierung nach Anspruch 1 oder 2, die weiters einen oder mehrere aus nichtionogenen Tensiden, Zinkionen und Glycin ausgewählte(n) Bestandteil(e) enthält.

4. Formulierung nach Anspruch 3, die weiters eine therapeutisch wirksame Menge von Insulin-artigem Wachstumsfaktor-1 umfasst.

5. Formulierung nach Anspruch 4, die weiters eine therapeutisch wirksame Menge von Insulin-artigem Wachstumsfaktor-Bindungsprotein umfasst.

6. Verwendung von Wachstumshormon-Bindungsprotein und Wachstumshormon zur Herstellung einer therapeutisch wirksamen Formulierung zur Stimulierung von auf Wachstumshormon ansprechendem Gewebe eines Säugetiers oder Vogels.

7. Formulierung, umfassend Wachstumshormon-Bindungsprotein, Wachstumshormon und ein nichtionogenes Tensid.

8. Formulierung, umfassend Wachstumshormon-Bindungsprotein, Wachstumshormon und Zink.

9. Formulierung nach Anspruch 8, die weiters eine Retard-Formulierung umfasst.

## Patentansprüche (Patentansprüche für folgende(n) Vertragsstaat(en): ES, GR)

1. Formulierung, die eine therapeutisch wirksame Menge an Wachstumshormon-Bindungsprotein und Wachstumshormon umfasst, zur Verwendung in einem Verfahren zur Stimulierung von auf Wachstumshormon ansprechendem Gewebe eines Säugetiers oder Vogels.

2. Formulierung nach Anspruch 1, worin die Dosis an Wachstumshormon und Wachstumshormon-Bindungsprotein etwa 1 µg/kg/Tag bis etwa 50 mg/kg/Tag beträgt.

3. Formulierung nach Anspruch 1 oder 2, die weiters einen oder mehrere aus nichtionogenen Tensiden, Zinkionen und Glycin ausgewählte(n) Bestandteil(e) enthält.

4. Formulierung nach Anspruch 3, die weiters eine therapeutisch wirksame Menge von Insulin-artigem Wachstumsfaktor-1 umfasst.

5. Formulierung nach Anspruch 4, die weiters eine therapeutisch wirksame Menge von Insulin-artigem Wachstumsfaktor-Bindungsprotein umfasst.

6. Verwendung von Wachstumshormon-Bindungsprotein und Wachstumshormon zur Herstellung einer therapeutisch wirksamen Formulierung zur Stimulierung von auf Wachstumshormon ansprechendem Gewebe eines Säugetiers oder Vogels.

7. Formulierung, umfassend Wachstumshormon-Bindungsprotein, Wachstumshormon und ein nichtionogenes Tensid.

8. Formulierung, umfassend Wachstumshormon-Bindungsprotein, Wachstumshormon und Zink.

9. Formulierung nach Anspruch 8, die weiters eine Retard-Formulierung umfasst.

10. Verfahren zur Herstellung einer Zusammensetzung zur Verwendung in einem Verfahren zur Stimulierung von auf Wachstumshormon ansprechendem Gewebe eines Säugetiers oder Vogels, wobei das Verfahren das Formulieren einer therapeutisch wirksamen Menge an Wachstumshormon-Bindungsprotein und Wachstumshormon umfasst.

11. Verfahren nach Anspruch 10, worin die Dosis an Wachstumshormon und Wachstumshormon-Bindungsprotein etwa 1 µg/kg/Tag bis etwa 50 mg/kg/Tag beträgt.

12. Verfahren nach Anspruch 10 oder 11, worin die Zusammensetzung weiters einen oder mehrere aus nichtionogenen Tensiden, Zinkionen und Glycin ausgewählte(n) Bestandteil(e) enthält.

13. Verfahren nach Anspruch 12, worin die Zusammensetzung weiters eine therapeutisch wirksame Menge von Insulin-artigem Wachstumsfaktor-1 umfasst.

14. Verfahren nach Anspruch 13, worin die Zusammensetzung weiters eine therapeutisch wirksame Menge von Insulin-artigem Wachstumsfaktor-Bindungsprotein umfasst.

15. Verfahren zur Herstellung einer Zusammensetzung, umfassend das Formulieren von Wachstumshormon-Bindungsprotein, Wachstumshormon und eines nichtionogenen Tensids.

16. Verfahren zur Herstellung einer Zusammensetzung, umfassend das Formulieren von Wachstumshormon-Bindungsprotein, Wachstumshormon und Zink.

17. Verfahren nach Anspruch 16, worin die Zusammensetzung weiters eine Retard-Formulierung umfasst.

## Revendications (Revendications pour l'(les) Etat(s) contractant(s) suivant(s): AT, BE, CH, LI, DE, DK, FR, GB, IT, LU, NL, SE)

1. Formulation comprenant une quantité thérapeutiquement efficace de protéine de liaison d'hormone de croissance et d'hormone de croissance, destinée à être utilisée dans une méthode pour stimuler les tissus sensibles à l'hormone de croissance d'un mammifère ou d'un oiseau.

2. Formulation suivant la revendication 1, dans laquelle la dose d'hormone de croissance et de protéine de liaison d'hormone de croissance est comprise dans l'intervalle d'environ 1 µg/kg/jour à environ 50 mg/kg/jour.

3. Formulation suivant la revendication 1 ou la revendication 2, contenant en outre un ou plusieurs ingrédients choisis entre les suivants : un agent tensio-actif non ionique, l'ion zinc et la glycine.

4. Formulation suivant la revendication 3, comprenant en outre une quantité thérapeutiquement efficace de facteur de croissance 1 analogue à l'insuline.

5. Formulation suivant la revendication 4, comprenant en outre une quantité thérapeutiquement efficace de protéine de liaison de facteur de croissance analogue à l'insuline.

6. Utilisation de la protéine de liaison d'hormone de croissance et d'hormone de croissance dans la production d'une formulation thérapeutiquement efficace pour stimuler les tissus sensibles à l'hormone de croissance d'un mammifère ou d'un oiseau.

7. Formulation comprenant à la fois la protéine de liaison d'hormone de croissance, l'hormone de croissance et un agent tensio-actif non ionique.

8. Formulation comprenant la protéine de liaison d'hormone de croissance, l'hormone de croissance et du zinc.

9. Formulation suivant la revendication 8, comprenant en outre une formulation à libération lente.

## Revendications (Revendications pour l'(les) Etat(s) contractant(s) suivant(s): ES, GR)

1. Formulation comprenant une quantité thérapeutiquement efficace de protéine de liaison d'hormone de croissance et d'hormone de croissance, destinée à être utilisée dans une méthode pour stimuler les tissus sensibles à l'hormone de croissance d'un mammifère ou d'un oiseau.

2. Formulation suivant la revendication 1, dans laquelle la dose d'hormone de croissance et de protéine de liaison d'hormone de croissance est comprise dans l'intervalle d'environ 1 µg/kg/jour à environ 50 mg/kg/jour.

3. Formulation suivant la revendication 1 ou la revendication 2, contenant en outre un ou plusieurs ingrédients choisis entre les suivants : un agent tensio-actif non ionique, l'ion zinc et la glycine.

4. Formulation suivant la revendication 3, comprenant en outre une quantité thérapeutiquement efficace de facteur de croissance 1 analogue à l'insuline.

5. Formulation suivant la revendication 4, comprenant en outre une quantité thérapeutiquement efficace de protéine de liaison de facteur de croissance analogue à l'insuline.

6. Utilisation de la protéine de liaison d'hormone de croissance et de l'hormone de croissance dans la production d'une formulation thérapeutiquement efficace pour stimuler les tissus sensibles à l'hormone de croissance d'un mammifère ou d'un oiseau.

7. Formulation comprenant à la fois la protéine de liaison d'hormone de croissance, l'hormone de croissance et un agent tensio-actif non ionique.

8. Formulation comprenant la protéine de liaison d'hormone de croissance, l'hormone de croissance et du zinc.

9. Formulation suivant la revendication 8, comprenant en outre une formulation à libération lente.

10. Procédé pour la production d'une composition destinée à être utilisée dans une méthode pour stimuler les tissus sensibles à l'hormone de croissance d'un mammifère ou d'un oiseau, procédé comprenant la formulation d'une quantité thérapeutiquement efficace de protéine de liaison d'hormone de croissance et d'hormone de croissance.

11. Procédé suivant la revendication 10, dans lequel la dose d'hormone de croissance et de protéine de liaison d'hormone de croissance est comprise dans l'intervalle d'environ 1 µg/kg/jour à environ 50 mg/kg/jour.

12. Procédé suivant la revendication 10 ou la revendication 11, dans lequel la composition contient en outre un ou plusieurs ingrédients choisis entre les suivants : un agent tensio-actif non ionique, l'ion zinc et la glycine.

13. Procédé suivant la revendication 12, dans lequel la composition comprend en outre une quantité thérapeutiquement efficace de facteur de croissance 1 analogue à l'insuline.

14. Procédé suivant la revendication 13, dans lequel la composition comprend en outre une quantité thérapeutiquement efficace de protéine de liaison de facteur de croissance analogue à l'insuline.

15. Procédé pour la production d'une composition comprenant la formulation de la protéine de liaison d'hormone de croissance, d'hormone de croissance et d'un agent tensio-actif non ionique.

16. Procédé pour la production d'une composition, comprenant la formulation de la protéine de liaison d'hormone de croissance, d'hormone de croissance et de zinc.

17. Procédé suivant la revendication 16, dans lequel la composition comprend en outre une formulation à libération lente.
